# EUROPEAN PATENT APPLICATION

(11) **EP 4 628 073 A1**
(43) Date of publication of application: **08.10.2025**
(21) Application number: 23896802.8
(22) Date of filing: 28.11.2023
(51) Int. Cl.: A61K 31/07, A61K 47/18, A61K 8/67, A61K 8/41, A61Q 19/00, A61P 17/00, A61P 17/10, A61P 17/06, A61P 31/10, A61P 35/00

(54) **COMPOSITION CONTAINING TRETINOIN, PREPARATION METHOD THEREFOR, AND USE THEREOF**

(30) Priority: 02.12.2022 CN 202211543235
(71) Applicant: NANJING INDETEK LABORATORY CO., LTD., Nanjing, Jiangsu 211106 (CN)
(72) Inventor: HU, Tao, Nanjing, Jiangsu 211106 (CN); LIU, Zhenyun, Nanjing, Jiangsu 211106 (CN); CHEN, Lei, Nanjing, Jiangsu 211106 (CN); LU, Xiqiang, Nanjing, Jiangsu 211106 (CN)
(74) Representative: Bjerkén Hynell KB
(86) International application number: PCT/CN2023/134849
(87) International publication number: WO 2024/114654

(57) **Abstract**

The present invention relates to a composition containing tretinoin, a preparation method therefor, and use thereof. The composition comprises (a) a tretinoin-alcoholamine compound complex, (b) a gel matrix, and (c) an oil phase component. The composition exhibits good component compatibility, comfort of use, stability, safety, and transdermal performance, thus having wide medicinal prospects.

## Description

The present application claims the benefit and priority of prior Chinese Patent Application No 202211543235.6, entitled "TRETINOIN-CONTAINING COMPOSITION, PREPARATION METHOD AND USE THEREOF" and filed with the China National Intellectual Property Administration on December 02, 2022, the disclosure of which is incorporated by reference herein in its entirety as part of the present application.

### TECHNICAL FIELD

The present disclosure belongs to the field of pharmaceutical chemistry, and in particular relates to a tretinoin-containing composition, a preparation method, and use thereof.

### BACKGROUND

Vitamin A and its metabolites play an important role in the human body and are currently widely used in the treatment of a variety of diseases. For example, tretinoin (molecular formula: C₂₀H₂₈O₂), an intermediate in the metabolism of vitamin A, mainly affects bone growth and promotes metabolic effects such as epithelial cell proliferation, differentiation, and keratolysis. Tretinoin is mainly used for the treatment of diseases such as acne vulgaris, hyperpigmentation, skin photoaging, psoriasis, ichthyosis, lichen planus, pityriasis rubra pilaris, keratosis follicularis, squamous cell carcinoma and melanoma. Oral tretinoin can also be used for the treatment of acute promyelocytic leukemia.

Tretinoin is fat-soluble and almost insoluble in water, and therefore has poor solubility in preparations and poor dissolution *in vivo,* thereby limiting the medicinal use of the preparations of tretinoin. In addition, due to the barrier function of the stratum corneum of the skin, only a small amount of tretinoin could pass through the barrier to play a role when a conventional topical tretinoin preparation is used, making it difficult to maintain an effective therapeutic concentration. Increasing the dosage of tretinoin, however, results in a greater cutaneous irritation, manifesting as varying degrees of inflammatory symptoms, such as redness, tingling, and edema. In view of tretinoin having good application prospects in the treatment of skin diseases, it is necessary to develop it into a medicinal form suitable for use in skin diseases.

It has been found through studies that the complex formed by tretinoin and an alcoholamine compound is in liquid form at ambient temperature, and has good water solubility/water dilution stability and skin penetration effect. On the basis of these findings regarding the complex, it is expected to further develop retinoin preparations with good medicinal properties.

### SUMMARY

To improve the technical problems existing in the prior art, in a first aspect, the present disclosure provides a composition including the components of:
(a) a tretinoin-alcoholamine compound complex;
(b) a gel matrix; and
(c) an oil phase component.

According to the present disclosure, the tretinoin-alcoholamine compound complex is composed of (or consists of) tretinoin and an alcoholamine compound.

In some embodiments, the composition includes:
(a) the tretinoin-alcoholamine compound complex;
(b) the gel matrix;
(c) the oil phase component; and
(d) an emulsifier.

According to some embodiments of the present disclosure, the (c) oil phase component is at least one selected from the group consisting of (c1) a greasy matrix, (c2) a co-solvent, and (c3) a penetration enhancer.

According to some embodiments of the present disclosure, the composition further includes (e) water.

In some embodiments, the composition includes:
(a) the tretinoin-alcoholamine compound complex;
(b) the gel matrix; and
(d) an emulsifier.

In some embodiments, the composition includes:
(a) the tretinoin-alcoholamine compound complex;
(b) the gel matrix;
(c2) a co-solvent;
(c3) a penetration enhancer; and
(d) an emulsifier.

In some embodiments, the composition includes:
(a) the tretinoin-alcoholamine compound complex;
(b) the gel matrix;
(c1) a greasy matrix;
(c2) a co-solvent; and
(d) an emulsifier.

In some embodiments, the composition includes:
(a) the tretinoin-alcoholamine compound complex;
(b) the gel matrix;
(c2) a co-solvent; and
(c3) a penetration enhancer.

In some embodiments, the composition includes:
(a) the tretinoin-alcoholamine compound complex;
(b) the gel matrix; and
(c3) a penetration enhancer.

According to some embodiments of the present disclosure, the gel matrix is selected carbomers, such as Carbopol 980, and Carbopol ETD2020.

According to some embodiments of the present disclosure, the co-solvent is selected from alcohols, and preferably is at least one selected from the group consisting of glycerol, ethanol, propylene glycol, and benzyl alcohol.

According to some embodiments of the present disclosure, the emulsifier is at least one selected from the group consisting of Tween 20, Tween 40, Tween 60, Tween 80, Span 20, Span 60, Span 80, a polyoxyethylene castor oil derivative, poloxamer, triton, polyethylene glycol glyceryl caprylate/caprate, polyethylene glycol stearate, polyethylene oxide-8 beeswax, lauroyl macrogolglyceride. In some embodiments, the emulsifier is selected from the group consisting of polyethylene glycol (32) stearate, and lauroyl macrogolglyceride (6); in some embodiments, the polyoxyethylene castor oil derivative is selected from the group consisting of polyoxyethylene castor oil and polyoxyl hydrogenated castor oil, such as polyoxyl 35 castor oil, polyoxyl 40 castor oil, polyoxyl 54 hydrogenated castor oil, and polyoxyl 100 hydrogenated castor oil.

According to some embodiments of the present disclosure, the greasy matrix is at least one selected from the group consisting of octadecanol, hexadecanol, stearic acid, a fatty acid ester; the fatty acid ester is at least one selected from isopropyl myristate, medium-chain triglyceride, glyceryl monooleate, glyceryl monolinoleate, propylene glycol dicaprylate/dicaprate, oleoyl polyoxyethylene glyceride, myristyl myristate, isopropyl palmitate, isopropyl linoleate, dodecyl benzoate, isostearyl isostearate, fatty acid lactylate, decyl oleate, and octyl palmitate.

According to some embodiments of the present disclosure, the penetration enhancer is at least one selected from the group consisting of glyceryl monocaprylate, laurocapram, diethylene glycol monoethyl ether, polyglycerol fatty acid ester, propylene glycol monocaprylate, and polyethylene glycol glyceryl caprylate caprate;
according to some embodiments of the present disclosure, the composition further includes one or more selected from the group consisting of an antioxidant, a preservative, a pH adjuster, a metal ion complexing agent, and a rheology modifier.

According to some embodiments of the present disclosure, the antioxidant is at least one selected from the group consisting of dibutyl hydroxytoluene, butylhydroxylanisole, vitamin C, vitamin E, and sodium metabisulfite; the preservative is at least one selected from the group consisting of benzyl alcohol, phenoxyethanol, sodium benzoate, methylparaben, ethylparaben, propylparaben, sodium methylparaben, and sodium propylparaben; the pH adjuster is at least one selected from the group consisting of triethanolamine and sodium hydroxide; the rheology modifier is at least one selected from the group consisting of Pemulen^{™} TR-1 NF polymer, Pemulen^{™} TR-2 NF polymer, Carbopol^{®} 1342 NF polymer, and Carbopol^{®} 5984 EP polymer.

According to some embodiments of the present disclosure, the metal ion complexing agent is at least one selected from the group consisting of ethylenediaminetetraacetic acid (EDTA), disodium ethylenediaminetetraacetic acid (EDTA-2Na), tetrasodium ethylenediaminetetraacetic acid (EDTA-4Na), disodium nitrilotriacetate, sodium tripolyphosphate, sodium hexametaphosphate, and tetrapotassium pyrophosphate, preferably EDTA or EDTA-2Na.

According to some embodiments of the present disclosure, the composition has a pH value of 4-7, such as 4.0, 4.5, 5.0, 5.5, 6.0, 6.5, 7.0, preferably 5.0-6.0.

In some embodiments, the composition is selected from the following ingredients:
(a) the tretinoin-alcoholamine compound complex;
(b) the gel matrix;
(c1) a greasy matrix, being at least one selected from the group consisting of octadecanol, and isopropyl myristate;
(c2) a co-solvent, being at least one selected from the group consisting of glycerol, ethanol, benzyl alcohol; and
(d) an emulsifier selected from Tween 80.

In some embodiments, the composition further optionally includes: one or more selected from the group consisting of Pemulen^{™} TR-1 NF, and dibutyl hydroxytoluene.

In some embodiments, the composition is selected from the following ingredients:
(a) the tretinoin-alcoholamine compound complex;
(b) the gel matrix;
(c2) a co-solvent selected from propylene glycol; and
(c3) a penetration enhancer selected from laurocapram;

The composition further optionally includes one or more selected from the group consisting of dibutyl hydroxytoluene, sodium methylparaben, and sodium propylparaben.

According to some embodiments of the present disclosure, the component (a) accounts for 0.01% to 5% (w/w), such as 0.01%, 0.05%, 0.10%, 0.11%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.50%, 0.60%, 0.70%, 0.80%, 0.90%, 1.0%, 1.1%, 1.2%, 1.3%, 1.4%, 1.5%, 1.6%, 1.7%, 1.8%, 1.9%, or 2.0% (w/w), of a total amount of the composition. In some embodiments, the component (a) accounts for 0.01%-0.5% (w/w) of the total amount of the composition.

According to some embodiments of the present disclosure, the component (b) accounts for 0.01% to 5% (w/w), such as 0.01%, 0.05%, 0.10%, 0.11%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.50%, 0.60%, 0.70%, 0.80%, 0.90%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, or 3.0% (w/w), of the total amount of the composition. In some embodiments, the component (b) accounts for 0.1%-2.0% (w/w) of the total amount of the composition.

According to some embodiments of the present disclosure, the component (c) accounts for 0.5% to 70.0% (w/w), such as 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 31.0%, 32.0%, 33.0%, 34.0%, 35.0%, 36.0%, 37.0%, 38.0%, 39.0%, 40.0%, 41.0%, 42.0%, 43.0%, 44.0%, 45.0%, 46.0%, 47.0%, 48.0%, 49.0%, or 50.0% (w/w), of the total amount of the composition. In some embodiments, the component (c) accounts for 1.0%-35.0% (w/w) of the total amount of the composition.

According to some embodiments of the present disclosure, the component (c1) accounts for 4.0% to 60.0% (w/w), such as 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 31.0%, 32.0%, 33.0%, 34.0%, 35.0%, 36.0%, 37.0%, 38.0%, 39.0%, 40.0%, 41.0%, 42.0%, 43.0%, 44.0%, 45.0%, 46.0%, 47.0%, 48.0%, 49.0%, or 50.0% (w/w), of the total amount of the composition. In some embodiments, the component (c1) accounts for 4.0%-30.0% (w/w) of the total amount of the composition. In some embodiments, under the condition that component (c1) is selected from two or more greasy matrices described above, each greasy matrix accounts for 4.0% to 20.0% (w/w), such as 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, or 20.0% (w/w), of the total amount of the composition.

According to some embodiments of the present disclosure, the component (c2) accounts for 0.5% to 25.0% (w/w), such as 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, or 30.0% (w/w), of the total amount of the composition. In some embodiments, the component (c2) accounts for 1.0%-20.0% (w/w) of the total amount of the composition. In some embodiments, under the condition that the component (c2) is selected from two or more co-solvents described above, each co-solvent accounts for 1.0% to 20.0% (w/w), such as 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, or 20.0% (w/w), of the total amount of the composition.

According to some embodiments of the present disclosure, the component (c3) accounts for 0.5% to 30.0% (w/w), such as 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, or 30.0% (w/w), of the total amount of the composition. In some embodiments, the component (c3) accounts for 1+.0%-25.0% (w/w) of the total amount of the composition.

According to some embodiments of the present disclosure, the component (d) accounts for 0.5% to 30.0% (w/w), such as 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, or 30.0% (w/w), of the total amount of the composition. In some embodiments, the component (d) accounts for 1.0%-20.0% (w/w) of the total amount of the composition.

According to some embodiments of the present disclosure, the antioxidant accounts for 0.01% to 5% (w/w), such as 0.01%, 0.05%, 0.10%, 0.11%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.50%, 0.60%, 0.70%, 0.80%, 0.90%, 1.0%, 1.1%, 1.2%, 1.3%, 1.4%, 1.5%, 1.6%, 1.7%, 1.8%, 1.9%, or 2.0% (w/w), of the total amount of the composition. In some embodiments, the antioxidant accounts for 0.01%-0.5% (w/w) of the total amount of the composition.

According to some embodiments of the present disclosure, the preservative accounts for 0.01% to 5% (w/w), such as 0.01%, 0.05%, 0.10%, 0.11%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.50%, 0.60%, 0.70%, 0.80%, 0.90%, 1.0%, 1.1%, 1.2%, 1.3%, 1.4%, 1.5%, 1.6%, 1.7%, 1.8%, 1.9%, or 2.0% (w/w), of the total amount of the composition. In some embodiments, the preservative accounts for 0.01%-0.2% (w/w) of the total amount of the composition.

According to some embodiments of the present disclosure, the rheology modifier accounts for 0.01% to 5% (w/w), such as 0.01%, 0.05%, 0.10%, 0.11%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.50%, 0.60%, 0.70%, 0.80%, 0.90%, 1.0%, 1.1%, 1.2%, 1.3%, 1.4%, 1.5%, 1.6%, 1.7%, 1.8%, 1.9%, or 2.0% (w/w), of the total amount of the composition. In some embodiments, the rheology modifier accounts for 0.01%-0.5% (w/w) of the total amount of the composition.

According to some embodiments of the present disclosure, the metal ion complexing agent accounts for 0.01% to 2.0% (w/w), such as 0.01%, 0.05%, 0.10%, 0.11%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.50%, 0.60%, 0.70%, 0.80%, 0.90%, 1.0%, 1.1%, 1.2%, 1.3%, 1.4%, 1.5%, 1.6%, 1.7%, 1.8%, 1.9%, or 2.0% (w/w), of the total amount of the composition. In some embodiments, the metal ion complexing agent accounts for 0.01%-0.5% (w/w) of the total amount of the composition.

According to some embodiments of the present disclosure, the alcoholamine compound is represented by formula I: 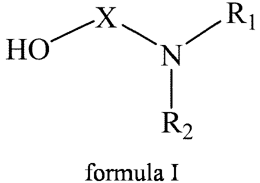 in formula I, X is selected from C₁₋₆ alkyl, and preferably is selected from the group consisting of -CH₂-CH₂-, -CH₂-CH₂-CH₂-, and 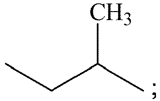 R₁ and R₂ each are independently selected from the group consisting of H, C₁₋₆ alkyl, and C₁₋₆ alkyl-OH.

According to some embodiments of the present disclosure, the formula I is selected from the group consisting of diethanolamine, triethanolamine, monoethanolamine (2-hydroxyethylamine), N-methyldiethanolamine, n-propanolamine, isopropanolamine, diisopropanolamine, triisopropanolamine, n-butanolamine, dibutanolamine, and isobutanolamine.

According to some embodiments of the present disclosure, a molar ratio of the alcoholamine compound to tretinoin is in a range of (1-100) : 1, such as 2 : 1, 2.5 : 1, 3 : 1, 3.5 : 1, 4 : 1, 4.5 : 1, 5 : 1, 5.5 : 1, 6 : 1, 6.5 : 1, 7 : 1, or (1-10) : 1, more preferably (1-5) : 1.

According to some embodiments of the present disclosure, the complex is in liquid form at room temperature. In the complex, the tretinoin may partially or entirely exist in anionic form, and the alcoholamine compound may partially or entirely exist in cationic form. In some embodiments, in the complex, the tretinoin partially exists in molecular form and/or the alcoholamine compound partially exists in molecular form.

In some embodiments, in the complex, the tretinoin exists in the form of [A⁻] as follows:

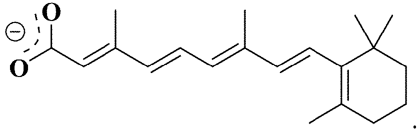

In some embodiments, in the complex, the alcoholamine compound exists in the form of [B⁺] as shown in the following formula II: 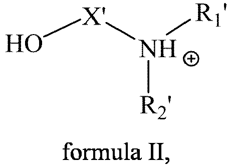 in formula II, X' is selected from C₁₋₆ alkyl, and preferably is selected from the group consisting of -CH₂-CH₂-, -CH₂-CH₂-CH₂-, and 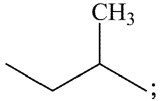 R₁' and R₂' each are independently selected from H, C₁₋₆ alkyl, and C₁₋₆ alkyl-OH.

According to some embodiments of the present disclosure, the formula II is selected from the group consisting of diethanolammonium cation, triethanolammonium cation, monoethanolammonium cation (i.e., 2-hydroxyethylammonium cation), N-methyldiethanolammonium cation, n-propanolammonium cation, isopropanolammonium cation, diisopropanolammonium cation, triisopropanolammonium cation, n-butanolammonium cation, dibutanolammonium cation, and isobutanolammonium cation.

In some embodiments, the complex is [A⁻] [B⁺]ₓ, where x is selected from 1-10, such as 1, 1.5, 2, 2.5, 3, 3.5, 4, 4.5, 5, 5.5, 6, 6.5, or 7.

According to some embodiments of the present disclosure, the complex is prepared by a preparation method including: reacting tretinoin with the alcoholamine compound.

According to some embodiments of the present disclosure, in the preparation method, a molar ratio of the alcoholamine compound to the tretinoin is in a range of (1-100) : 1, such as 2 : 1, 2.5 : 1, 3 : 1, 3.5 : 1, 4 : 1, 4.5 : 1, 5 : 1, 5.5 : 1, 6 : 1, 6.5 : 1, 7 : 1, or (1-10) : 1, more preferably (1-5) : 1.

According to some embodiments of the present disclosure, the reaction reagent for the reaction is in water, or an organic solvent, or a mixed solvent of an organic solvent and water, where the organic solvent is selected from one of or a combination of at least two of ethanol, methanol, and acetone. Preferably, the organic solvent is ethanol.

According to some embodiments of the present disclosure, a volume ratio of the organic solvent to the water in the mixed solvent is 5 : 95 to 95 : 5. Preferably, the volume ratio of the organic solvent to the water in the mixed solvent is 40 : 60 to 95 : 5.

According to some embodiments of the present disclosure, the reaction is performed by means of dry milling.

According to some embodiments of the present disclosure, the ratio of the tretinoin to the total amount of the reaction reagent is 1 g : 5-150 mL, preferably 1 g : 5-20 mL.

According to some embodiments of the present disclosure, the preparation method includes the steps of:
(1) adding a reaction reagent to the tretinoin, and stirring for mixing;
(2) adding a reaction reagent to the alcoholamine compound, and stirring for dilution;
(3) adding a diluted solution obtained in step (2) to a mixture obtained in step (1).

According to some embodiments of the present disclosure, in step (1), the ratio of the tretinoin to the amount of the reaction agent is 1 g : 5-100 mL, preferably 1 g : 5-20 mL; in step (2), the amount ratio of the alcoholamine compound to the reaction agent is 1 g : 0.5-50 mL, preferably 1 g : 0.5-5 mL, more preferably 1 g : 0.5-1 mL.

According to some embodiments of the present disclosure, the preparation method further includes the steps of removing the reaction reagent after completion of the reaction, and performing vacuum drying.

In a third aspect, the present disclosure provides a method for preparing the composition, which includes the steps of:
mixing the component (a), the component (b), the component (c), and stirring until uniform;
optionally, adding other auxiliary ingredients during the mixing.

In a fourth aspect, the present disclosure provides use of the composition in the preparation of a preparation including, but not limited to, pharmaceutical preparations, cosmetics, care products, beauty products.

Preferably, the pharmaceutical preparation is used for the treatment of skin diseases, such as acne, hyperpigmentation, skin photoaging, psoriasis, ichthyosis, lichen planus, pityriasis rubra pilaris, keratosis follicularis, squamous cell carcinoma and melanoma.

According to some embodiments of the present disclosure, the preparation is administered topically via the skin, for example selected from creams, patches, ointments, cream preparations, emulgel preparations, gels, sprays, etc., or orally (i.e., as an oral preparation), for example, selected from tablets, granules, capsules, oral liquid preparations, pills, suspensions, dripping pills, etc. The preparation further includes a physiologically acceptable carrier (e.g., a biocompatible material), for example, optionally including surfactants, excipients, humectants, emulsification accelerators, suspending agents, salts or buffers for osmotic pressure adjustment, colorants, flavoring agents, stabilizers, bactericides, preservatives or other conventional supplements. Preferably, the preparation is a cream, emulgel, or gel preparation.

According to some embodiments of the present disclosure, the route of administration of the preparation includes, but is not limited to, gastrointestinal or parenteral administration; wherein the gastrointestinal administration may be oral administration; the parenteral administration may be transdermal administration, etc.

### BENEFICIAL EFFECTS OF THE PRESENT DISCLOSURE

The composition containing a tretinoin-alcoholamine compound complex according to the present disclosure exhibits the preparation properties such as good component compatibility, comfort of use, stability, safety, and transdermal performance, thus having wide medicinal prospects.

### BRIEF DESCRIPTION OF THE DRAWINGS

FIG. 1 shows the proton nuclear magnetic resonance spectrum (HNMR) spectrum of tretinoin.
FIG. 2 shows the HNMR spectrum of diethanolamine.
FIG. 3 shows the HNMR spectrum of the complex 2[DEA][RA].
FIG. 4 shows the infrared (IR) spectra of tretinoin, diethanolamine, the complex 2[DEA][RA].
FIG. 5 shows the IR spectra of tretinoin-diethanolamine complexes with different molar ratios ([RA] : [DEA] = 1 : 2, 1 : 2.5, 1 : 3, 1 : 4).
FIG. 6 shows the Raman spectra of tretinoin, diethanolamine, the complex 2[DEA][RA].
FIG. 7 shows the dynamic moisture sorption (DVS) of the complex 2[DEA][RA].
FIG. 8 shows the PLM images of the complex 2[DEA][RA] before and after DVS.
FIG. 9 shows the differential scanning calorimetry (DSC) diagram of the complex 2[DEA][RA].
FIG. 10 shows the mDSC of the complex 2[DEA][RA].
FIG. 11 shows the properties of the complexes 2[DEA][RA] with different water contents.
FIG. 12 shows the liquid nitrogen freezing test process of the complex 2[DEA][RA].
FIG. 13 shows the HNMR spectrum of the complex 2.5[DEA][RA].
FIG. 14 shows the HNMR spectrum of the complex 3[DEA][RA].
Panel (a) of FIG. 15 shows that the complex 7[TEA][RA] could not be formed (precipitate observed, turbid state); panel (b) of FIG. 15 shows that the complex 8[TEA][RA] could be formed (reddish-brown transparent liquid).
FIG. 16 shows the transdermal permeation test results of the preparation according to an embodiment of the present disclosure.

### DETAILED DESCRIPTION OF THE EMBODIMENTS

The technical solutions of the present disclosure will be described in further detail below in conjunction with specific examples. It should be understood that the following examples illustrate and explain the present disclosure by way of example only and should not be construed as limiting the scope of the present disclosure. All techniques achieved based on the above content of the present disclosure be encompassed within the scope of the present disclosure.

Unless otherwise specified, raw materials and reagents used in the following examples are commercially available or could be prepared by known methods.

### Instruments and test conditions:

| Test item | Equipment | Test conditions |
|---|---|---|
| Proton nuclear magnetic resonance spectroscopy | Bruker 400M NMR Spectrometer | Solvent: DMSO-d6 |
| Proton nuclear magnetic resonance spectroscopy | Bruker 600M NMR Spectrometer | Solvent: DMSO-d6 |
| Infrared spectroscopy | Brucker Vertex 70 | Attenuated total reflection (ATR) mode, collected at room temperature, beam splitter of KBr, test in a wavenumber range of 4000 to 600 cm⁻¹, a resolution of 4 cm⁻¹ |
| Infrared spectroscopy | ThermoFisher Nicolet FT-IR | Attenuated total reflection (ATR) mode, collected at room temperature, beam splitter of KBr, test in a wavenumber range of 4000 to 600 cm⁻¹, a resolution of 4 cm⁻¹ |
| Raman spectroscopy | HORIBA HR800 | Laser wavelength of 633 nm, grating wavelength of 600 nm |
| Dynamic moisture sorption test | SMS Intrinsic dynamic moisture sorption analyzer | Temperature: 25 °C, gas flow: nitrogen, minimum equilibration time: 10 min, maximum equilibration time: 180 min, resolution: 0.002%/min, humidity range: 0%RH-95%RH-0%RH |
| Polarized light microscopy | Carl Zeiss Axio Scope A1 | Takeing photograph at room temperature |

Unless otherwise specified, the percentage "%" in the examples below represents the mass percentage.

### Example 1 Preparation of a tretinoin-diethanolamine complex

**1.1 Materials and reagents:** Tretinoin (RA) (AR, Shanghai Aladdin Bio-Chem Technology Co., Ltd., China), diethanolamine (DEA) (AR, Sinopharm Chemical Reagent Co., Ltd., China), ethanol (AR, Sinopharm Chemical Reagent Co., Ltd., China).
**1.2 Process for preparing the tretinoin-diethanolamine complex**
**1.2.1 Preparation method I**

### Prescription

| Material | Amount (molar ratio of DEA to RA of 2 : 1) | Solvent | Amount of solvent |
|---|---|---|---|
| Tretinoin (RA) | 6.0 g | Ethanol | 30 mL to 60 mL |
| Diethanolamine (DEA) | 4.2 g | Ethanol | 5 mL |

(1) The prescribed amount of tretinoin was weighed and placed in a round-bottom flask, ethanol was added thereto, and stirring was performed for mixing, wherein in the resulting mixture, the tretinoin could not be completely dissolved, forming a suspension, and the round-bottom flask was wrapped with an aluminum foil for protection from light.
(2) The prescribed amount of diethanolamine was weighed and placed in an aluminum foil-wrapped container, and diluted by adding ethanol with stirring.

The diluted diethanolamine obtained in step (2) was added dropwise to the mixture obtained in step (1) with stirring; and the aluminum foil-wrapped container was washed with about 5 mL of ethanol, and the resulting washing solution was added into the round-bottom flask, followed by stirring.

After the dropwise addition was completed, the resulting solution should appear as an orange-red, clear liquid. Stirring was continued for 4 h.

The solvent was removed using a rotary evaporator: vacuum pressure (gauge reading): -0.095 Mpa; programmed temperature gradient was adopted to prevent ethanol bumping; rotary evaporation was conducted at 45 °C for 1 h, after raising temperature to 60 °C, rotary evaporation was conducted at 60 °C for 2 h. A reddish-brown viscous liquid was obtained, which was pipetted while hot, and then subjected to vacuum drying in a vacuum oven at 60 °C for 48 h. The resulting sample was sealed and stored in the dark.

### 1.2.2 Preparation method II

### Prescription:

| Material | Amount (molar ratio of DEA to RA of 2 : 1) | Solvent | Amount of solvent |
|---|---|---|---|
| Tretinoin (RA) | 6.0 g | Ethanol : water = 50 : 50 | 50 mL |
| Diethanolamine (DEA) | 4.2 g | Ethanol : water = 50 : 50 | 5 mL |

### Process:

The prescribed amount of tretinoin was weighed and placed in a round-bottom flask, ethanol/water was added thereto, and stirring was performed for mixing, wherein in the resulting mixture, the tretinoin could not be completely dissolved, forming a suspension, and the round-bottom flask was wrapped with an aluminum foil for protection from light.

The prescribed amount of diethanolamine was weighed and placed in an aluminum foil-wrapped container, and diluted by adding ethanol/water with stirring.

The diluted diethanolamine was added dropwise to the above round-bottom flask containing the tretinoin mixture with stirring; and the aluminum foil-wrapped container was washed with about 5 mL of ethanol/water, and the resulting washing solution was added into the round-bottom flask, followed by stirring. After the dropwise addition was completed, the resulting solution should appear as an orange-red, clear liquid. Stirring was continued for 4 h. The solvent was removed using a rotary evaporator. A reddish-brown viscous liquid was obtained, which was pipetted while hot, and then subjected to vacuum drying in a vacuum oven at 60 °C for 48 h. The resulting sample was sealed and stored in the dark.

### 1.2.3 Preparation method III

### Prescription:

| Material | Amount | Solvent | Amount of solvent |
|---|---|---|---|
| Tretinoin (RA) | 6.0 g | \ | \ |
| Diethanolamine (DEA) | 4.2 g | Water | 50 ml |

### Process:

The prescribed amount of diethanolamine was weighed and placed in a round-bottom flask, wherein the round-bottom flask was wrapped with an aluminum foil for protection from light.

The prescribed amount of water was added into the round-bottom flask, and stirring was performed until uniform. The prescribed amount of tretinoin was weighed, and the powder was carefully added into the round-bottom flask in batches with stirring. After stirring until the solution was clear, stirring was continued for 4 h. The solvent was removed using a rotary evaporator. A reddish-brown viscous liquid was obtained, which was pipetted while hot, and then subjected to vacuum drying in a vacuum oven at 60 °C for 48 h. The resulting sample was sealed and stored in the dark.

### 1.2.4 Preparation method IV

### Prescription:

| Material | Amount (molar ratio of DEA to RA of 3 : 1) |
|---|---|
| Tretinoin (RA) | 6.0 g |
| Diethanolamine (DEA) | 6.3 g |

### Process:

Pay attention to protecting from light during the test. The prescribed amount of tretinoin was weighed and placed in a crucible; the prescribed amount of diethanolamine was then added thereto. The two substances were carefully mixed until uniform by using a narrow pestle. The crucible was heated in a heating mantle, where the temperature was maintained at 60 °C to 80 °C. Grinding was continued with stirring until a reddish-brown viscous liquid was obtained. The reddish-brown viscous liquid was transferred while hot to a light-protected container, cooled and stored with sealed.

### 1.3 Preparation of tretinoin-diethanolamine complexes with different ratios

Referring to the preparation process in 1.2.1, complexes were prepared using tretinoin and diethanolamine at different ratios (as shown in the table below). The results showed that tretinoin and diethanolamine could form complexes at molar ratios of 2 : 1 to 10 : 1 (all reddish-brown clear liquids at room temperature).

**Table A-1 Complexes formed by tretinoin and diethanolamine at different ratios**

| **Name** | **Molar ratio [DEA] : [RA]** | **State at room temperature** |
|---|---|---|
| 2[DEA][RA] | 2: 1 | Reddish-brown clear liquid |
| 2.5 [DEA] [RA] | 2.5 : 1 | |
| 3[DEA][RA] | 3 : 1 | |
| 4[DEA][RA] | 4: 1 | |
| 5[DEA][RA] | 5 : 1 | |
| 6[DEA][RA] | 6 : 1 | |
| 7[DEA][RA] | 7 : 1 | |
| 8[DEA][RA] | 8 : 1 | |
| 9[DEA][RA] | 9 : 1 | |
| 10[DEA][RA] | 10 : 1 | |

### 1.4 Characterization of tretinoin-diethanolamine complex

Experimental object: By detecting and analyzing the raw materials and the tretinoin-diethanolamine complexes via proton nuclear magnetic resonance spectroscopy, infrared spectroscopy and Raman spectroscopy, it can be judged that the substance formed from tretinoin and diethanolamine is not a simple physical mixture. Instead, intermolecular interactions (including ionic bonds and hydrogen bonds) exist between tretinoin and diethanolamine, which constitute the chemical basis for the particular physicochemical properties of the tretinoin complex.

### 1.4.1 Proton nuclear magnetic resonance spectroscopy (HNMR)

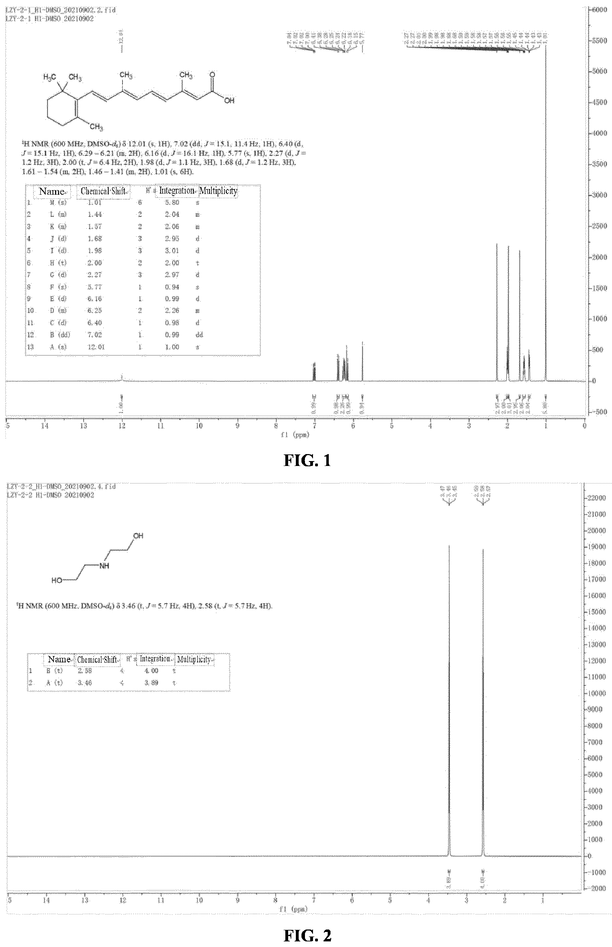

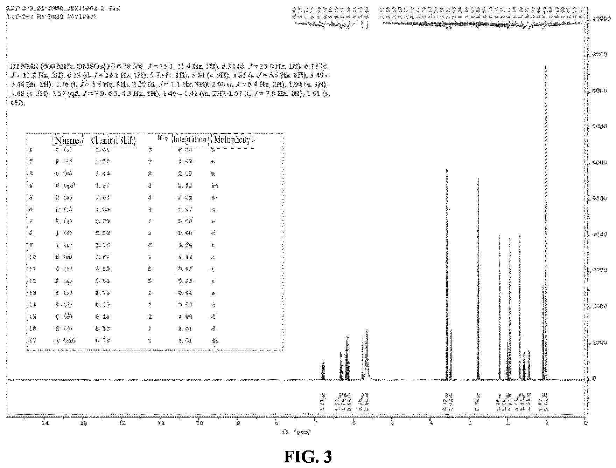

The tretinoin, diethanolamine and the prepared tretinoin-diethanolamine complex (2[DEA][RA]) were subjected to proton nuclear magnetic resonance spectroscopy, as shown in FIG. 1 to FIG. 3 and the following table:

**Table A-2 Chemical shift of characteristic hydrogen atom**

| **Molecules** | **Hydrogen position** | **Original chemical shift** | **Chemical shift in the complex** |
|---|---|---|---|
| Tretinoin | Hydrogen in carboxyl | 12.01 | Disappeared |
| | C-2 | 7.02 | 6.78 |
| Diethanolamine | C-1/C-1' | 2.58 | 2.76 |
| | C-2/C-2' | 3.46 | 3.56 |

The results show that the active hydrogen signal of tretinoin disappear in the HNMR of 2[DEA][RA], indicating that the tretinoin interacts with diethanolamine. The hydrogen atom ortho to the carboxyl of tretinoin exhibits an upfield shift in chemical shift (7.02→6.78), indicating that the tretinoin acts as a proton donor in the complex system, leading to anionization with the charge center localized on the carboxyl oxygen atom. Both pairs of methylene hydrogen on diethanolamine exhibit a downfield shift in chemical shift (2.58→2.76, 3.46→3.56, respectively), indicating that the diethanolamine acts as a proton acceptor in the complex system. Specifically, the lone pair of electrons on the N atom bound to a free proton, leading to protonation with the charge center localized on the N atom.

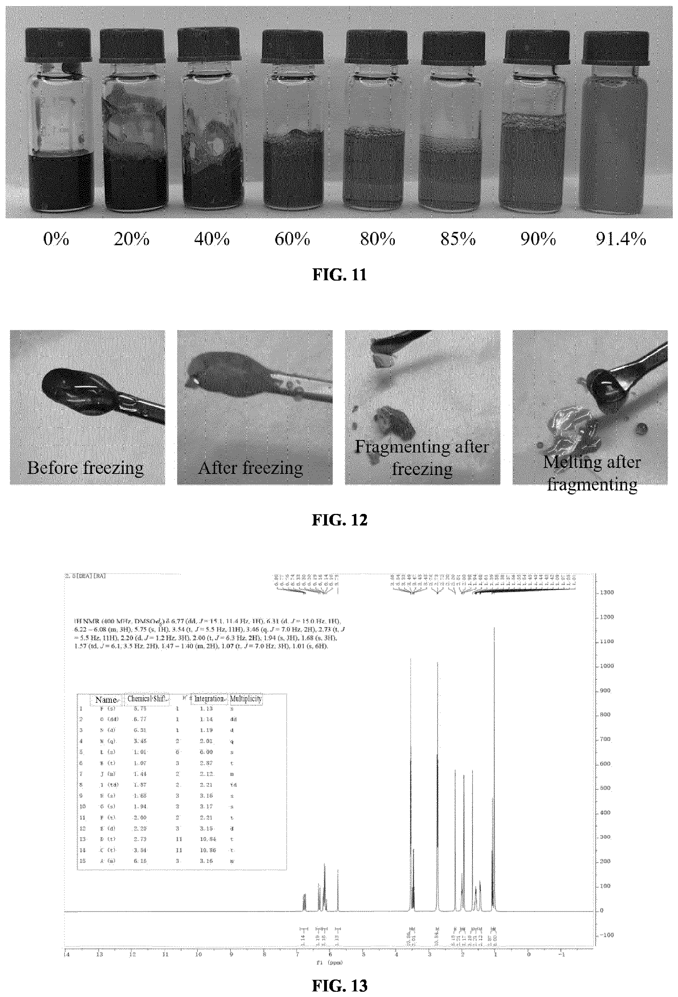

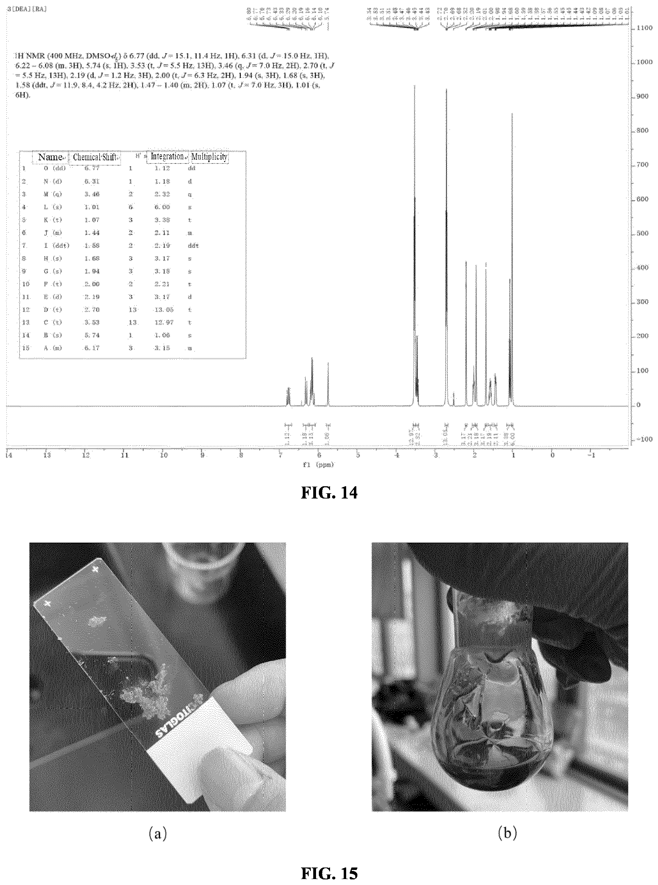

FIG. 13 and FIG. 14 show the HNMR spectra of complexes 2.5[DEA][RA] and 3[DEA][RA], respectively, also exhibiting the same characteristic chemical shift changes as those for 2[DEA][RA]. It can be seen that the same intermolecular interactions occur between diethanolamine at higher proportions and tretinoin, and liquid complexes that are stable at room temperature could also be formed.

### 1.4.2 Infrared spectroscopy (IR)

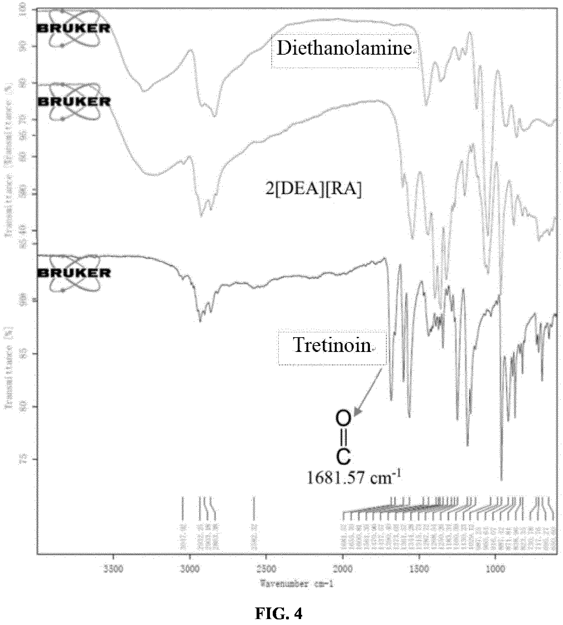

The tretinoin, diethanolamine and the prepared 2[DEA][RA] were identified by IR (as shown in FIG. 4). The results show that: compared with the diethanolamine, the peak of diethanolamine in 2[DEA][RA] exhibits no significant shift, and compared with the tretinoin, the characteristic frequency of C=O (1681.57 cm⁻¹) of tretinoin in 2[DEA][RA] shifts to a lower wavenumber, indicating that there are intermolecular interactions between the tretinoin and the diethanolamine.

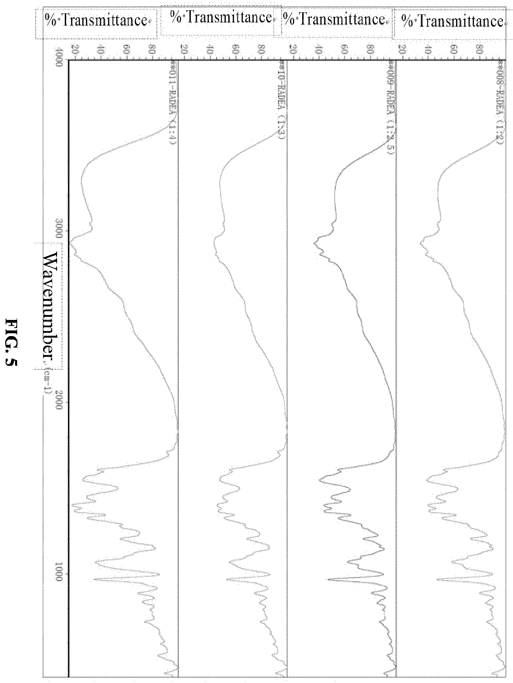

Complexes with different molar ratios were prepared according to molar ratios (RA : DEA = 1 : 2, 1 : 2.5, 1 : 3, 1 : 4); and the infrared spectra of the complexes with different molar ratios were consistent (as shown in FIG. 5), and all show a shift of the C=O characteristic frequency (1682 cm⁻¹) to a lower wavenumber, indicating that there are intermolecular interactions between the tretinoin and the diethanolamine.

### 1.4.3 Raman spectroscopy

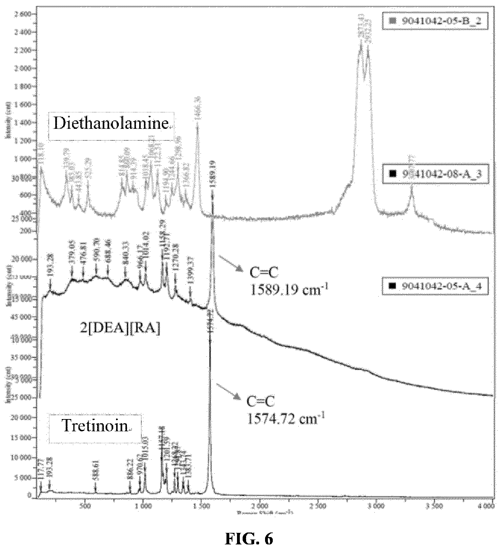

The tretinoin, diethanolamine and the prepared 2[DEA][RA] were identified by Raman spectroscopy (as shown in FIG. 6). The results show that: the Raman spectrum of 2[DEA][RA] is significantly different from that of the diethanolamine, and compared with the tretinoin, 2[DEA][RA] shows a shift of the C=C characteristic frequency (1574.72 cm⁻¹) to a higher wavenumber (1589.19 cm⁻¹), indicating that there are interactions between the tretinoin and the diethanolamine.

### Example 2 Preparation of tretinoin-triethanolamine complex

Referring to the preparation process in 1.2 in Example 1, diethanolamine was replaced with triethanolamine, and complexes were prepared using tretinoin and triethanolamine at different ratios (as shown in the table below). The results showed that the complexes were formed at molar ratios of tretinoin to triethanolamine of 1 : 8, 1 : 9, and 1 : 10, and it was difficult to form complexes in liquid form at molar ratios of 1 : (1-7) (see FIG. 15).

**Table B-1 Tretinoin-triethanolamine complexes with different ligand ratios**

| **Name** | **Molar ratio (TEA:RA)** | **State at room temperature** |
|---|---|---|
| 7[TEA][RA] | 7 : 1 | Turbid liquid with suspended yellow solid (RA precipitation). |
| 8[TEA][RA] | 8 : 1 | Reddish-brown clear liquid |
| 9[TEA][RA] | 9 : 1 | Reddish-brown clear liquid |
| 10[TEA][RA] | 10 : 1 | Reddish-brown clear liquid |

### Example 3 Study on general physicochemical properties of tretinoin-alcoholamine compound complexes

The physicochemical properties of 2[DEA][RA] prepared according to 1.2.1 were studied as follows:

### 3.1 Moisture and residual solvent

Three batches of 2[DEA][RA] were tested using a Karl Fischer moisture tester. It could be seen from the results that the moisture and residual solvent in the prepared complexes were easily removed.

**Table C-1 Moisture contents of tretinoin complexes**

| **Batch** | **Batch 1** | **Batch 2** | **Batch 3** |
|---|---|---|---|
| **Moisture content** | 0.38% | 0.44% | 0.41% |

### 3.2 Dynamic moisture sorption (DVS)

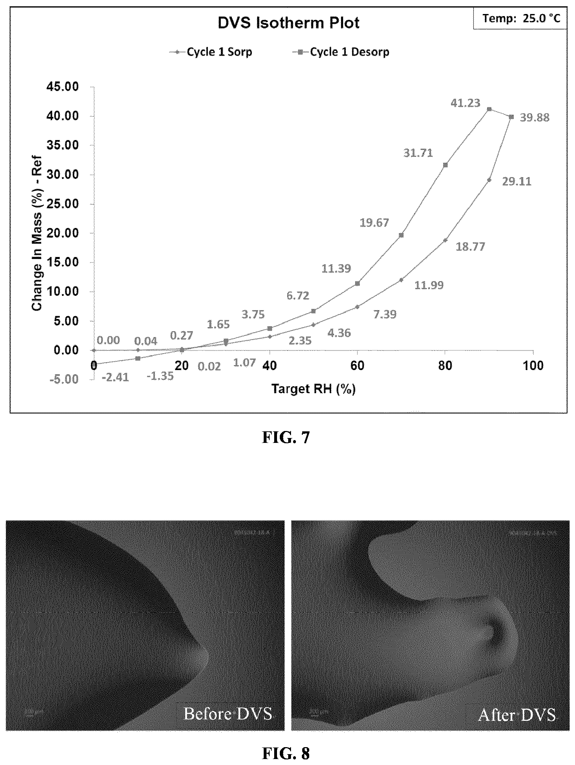

The DVS results (see FIG. 7) show that 2[DEA][RA] exhibits certain hygroscopic properties, while the PLM results (see FIG. 8) show that there are no crystalline particles in 2[DEA][RA] before and after DVS, indicating that the complex has a low risk of tretinoin precipitation under ambient humidity and could stably retain its liquid form.

### 3.3 Differential scanning calorimetry (DSC)

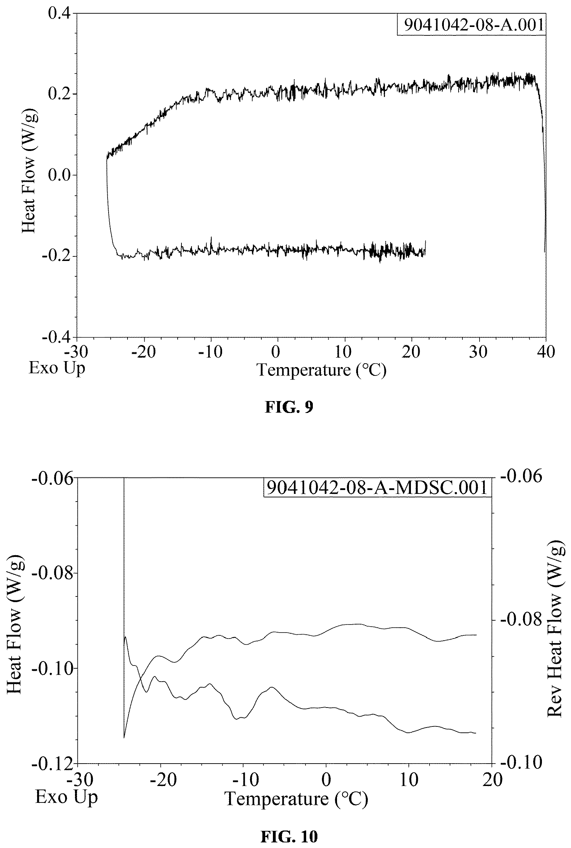

The DSC results (see FIG. 9) show that 2[DEA][RA] exhibits no exothermic or endothermic signals within the temperature range of -25 °C to 40 °C, indicating the absence of a freezing point of the complex in this temperature range. The mDSC results (see FIG. 10) show that no glass transition temperature is observed for the complex in the range of -25 °C to 25 °C, indicating that the complex could retain a stable liquid form within this temperature range, without the risk of solidification and precipitation.

### Example 4 Water solubility/water dilution stability of tretinoin complexes

### 4.1 Experimental object

According to the 2020 edition of Chinese Pharmacopoeia (Part II), tretinoin is extremely insoluble in water. Upon a review of literatures, it can be seen that the solubility of tretinoin in water is only 1.06*10⁻⁶ mol/L (Ascenso A, Guedes R, Bernardino R, et al. Complexation and full characterization of the tretinoin and dimethyl-β-cyclodextrin complex. AAPS PharmSciTech. 2011; 12(2):553-563. doi:10.1208/s12249-011-9612-3). The poor solubility of tretinoin poses great challenges for pharmaceutical processes. In contrast, tretinoin complexes have certain water solubility, which facilitates the manufacture of preparations.

### 4.2 Experimental process

2[DEA][RA] was accurately weighed, and subjected to vortex mixing with water at different proportions, and the appearances of the complexes with different water contents were observed; for the water content exceeding 90%, a small amount of the water was added multiple times by using a microsyringe with continuous vortex mixing until the solution appeared turbid. Weighing was performed, and calculation was performed to obtain the limiting water content of the complexes.

### 4.3 Results and discussion

As shown in FIG. 11, 2[DEA][RA] exhibits water miscibility within the water content range of 0 to 91.4% (w/w) (clear and transparent liquid in the bottle, with water content ≤ 90%), and when the water content is too high (> 91.4%), the hydrogen bonds in the water disrupt the complex system, resulting in the precipitation of tretinoin solid (turbid liquid in the bottle, with water content of 91.4%). The water dilution stability of complexes with other molar ratios was tested. The results are shown in the table below. With the increase of the proportion of DEA, the water dilution stability of the tretinoin-diethanolamine complexes increases.

**Table D-1 Water dilution stability of tretinoin-diethanolamine complexes with different ratios**

| **[RA] : [DEA]** | **1:2** | **1 : 2.5** | **1:3** | **1 : 4** |
|---|---|---|---|---|
| **Maximum water content** | 91.4% | 92.5% | 93.7% | 94.1% |

### Example 5 Crystallization induction test of tretinoin complexes

### 5.1 Experimental object

In the practice of chemical synthesis processes, compounds often become amorphous or supercooled liquids during recrystallization, and also exhibit glassy or clear oily morphologies. These characteristics are similar to those of the complexes of the present disclosure. However, this state is not stable, and transforms into the stable crystalline state upon external condition changes or crystal seed introduction. Thus, in order to exclude this possibility and further show that the complex prepared according to the present disclosure has a stable liquid form, a series of induction challenge tests are designed in order to obtain a solid form of the complex by a variety of means for promoting crystallization, such as evaporation test, stirring test and cooling test; and if the solid form could not be obtained, the liquid form of the resulting complex could be considered to be its stable form.

### 5.2 Evaporation test

Whether a solid substance was formed after complete solvent evaporation was investigated in different solvent systems, in the presence or absence of polymers, and at different temperatures, wherein addition of polymers was to add "crystal nucleus" to the system to compare the effect of the presence or absence of crystal nucleus, and different temperatures would affect the solvent evaporation rate and the crystallization behavior of the substance.

About 80 mg of 2[DEA][RA] was dissolved in the corresponding solvent to obtain a clear solution, the clear solution was divided equally into 4 groups, the solutions were subjected to different treatments, with a polymer acting as the "crystal nucleus": without the polymer; adding polymer B; adding polymer C, followed by evaporation at 5 °C or 50 °C for about 4 days. The states of the samples were observed, and no solid form substance was observed except for the groups with added polymer. The results are shown in the table below.

**Table E-1 Evaporation test results**

| No. | Solvent system | Mass | Volume | Temperature | Polymer | Results |
|---|---|---|---|---|---|---|
| Evaporation test-Al | acetone | 83.5 mg | 0.6 mL | 5 °C | No addition | Yellow-brown viscous substance |
| Evaporation test-A2 | | | | | Polymer B | Yellow-brown viscous substance |
| Evaporation test-A3 | | | | 50 °C | No addition | Reddish-brown viscous substance |
| Evaporation test-A4 | | | | | Polymer C | Reddish-brown viscous substance |
| Evaporation test-B1 | 2-MeTHF | 79.9 mg | 0.4 mL | 5 °C | No addition | Yellow-brown viscous substance |
| Evaporation test-B2 | | | | | Polymer B | Yellow-brown viscous substance |
| Evaporation test-B3 | | | | 50 °C | No addition | Reddish-brown viscous substance |
| Evaporation test-B4 | | | | | Polymer C | Reddish-brown viscous substance |
| Evaporation test-C1 | MeOH/THF (5 : 1, v/v) | 80.0 mg | 0.4 mL | 5 °C | No addition | Yellow-brown viscous substance |
| Evaporation test-C2 | | | | | Polymer B | Yellow-brown viscous substance |
| Evaporation test-C3 | | | | 50 °C | No addition | Reddish-brown viscous substance |
| Evaporation test-C4 | | | | | Polymer C | Reddish-brown viscous substance |
| Evaporation test-D1 | EtOAc/AC N (10 : 1, v/v) | 79.6 mg | 0.4 mL | 5 °C | No addition | Yellow-brown viscous substance |
| Evaporation test-D2 | | | | | Polymer B | Yellow-brown viscous substance |
| Evaporation test-D3 | | | | 50 °C | No addition | Reddish-brown viscous substance |
| Evaporation test-D4 | | | | | Polymer C | Reddish-brown viscous substance |

Polymer B: a mixture composed of five polymers in equal mass ratios: polycaprolactone (PCL), polyethylene glycol (PEG), polymethyl methacrylate (PMMA), Stearyl acrylate (SA), and hydroxyethyl cellulose (HEC).

Polymer C: a mixture composed of four polymers in equal mass ratios: polystyrene, polytetrafluoroethylene, poly(tribromostyrene), and poly(vinyl stearate).

### 5.3 Stirring test

0.3 mL of the respective solvent was added to about 20 mg of 2[DEA][RA] at room temperature (about 22 °C), and after 3 days of stirring at the respective temperature, a clear solution was obtained or oiling-out phenomenon was observed, without solid form substance obtained. The results are shown in the table below:

**Table E-2 Stirring test results**

| No. | Solvent system | Mass | Temperature | Results |
|---|---|---|---|---|
| Stirring test-A1 | EtOH | 20.8 mg | 5 °C | Initially clear, still clear after 3 days of stirring |
| Stirring test-A2 | | 21.9 mg | -20 °C | Initially clear, still clear after 3 days of stirring |
| Stirring test-B1 | DCM | 21.8 mg | 5 °C | Initially clear, still clear after 3 days of stirring |
| Stirring test-B2 | | 21.5 mg | -20 °C | Initially clear, still clear after 3 days of stirring |
| Stirring test-C1 | IPAc | 21.4 mg | 5 °C | Initially clear, oiling-out phenomenon occurred after 3 days of stirring |
| Stirring test-C2 | | 19.9 mg | -20 °C | Initially clear, oiling-out phenomenon occurred after 3 days of stirring |
| Stirring test-D1 | toluene | 21.9 mg | 5 °C | Initially clear, still clear after 3 days of stirring |
| Stirring test-D2 | | 20.0 mg | -20 °C | Initially clear, still clear after 3 days of stirring |
| Stirring test-E1 | MEK/AC N (3 : 2, v/v) | 19.9 mg | 5 °C | Initially clear, still clear after 3 days of stirring |
| Stirring test-E2 | | 20.7 mg | -20 °C | Initially clear, still clear after 3 days of stirring |
| Stirring test-F1 | THF/n-he ptane (1 : 2, v/v) | 20.4 mg | 5 °C | Initially oiling-out, still oiling-out after 3 days of stirring |
| Stirring test-F2 | | 20.7 mg | 50 °C | Initially oiling-out, still oiling-out after 3 days of stirring |
| Stirring test-G1 | acetone/H 2O (2 : 1, v/v) | 20.7 mg | 5 °C | Initially clear, still clear after 3 days of stirring |
| Stirring test-G2 | | 21.1 mg | -20 °C | Initially clear, still clear after 3 days of stirring |

### 5.4 Cooling test

About 40 mg of 2[DEA][RA] was dissolved in the respective solvent at 50 °C, a clear solution was obtained and divided equally in 2 groups. After cooling according to the respective procedure, and equilibrating at low temperature for 3 days, no solid form substance was obtained. The results are shown in the table below:

**Table E-3 Cooling test results**

| No. | Solvent system | Mass | Volume | Temperature | Results |
|---|---|---|---|---|---|
| Cooling test-A1 | n-propanol | 40.3 mg | 0.2 mL | 50 °C→5 °C | Clear |
| Cooling test-A2 | | | | 50 °C→-20 °C | Clear |
| Cooling test-C1 | 1,4-dioxane | 41.9 mg | 0.2 mL | 50 °C→5 °C | Clear |
| Cooling test-C2 | | | | 50 °C→-20 °C | Frozen, yellow opaque* |
| Cooling test-D1 | DMSO | 42.2 mg | 0.2 mL | 50 °C→5 °C | Clear |
| Cooling test-D2 | | | | 50 °C→-20 °C | Frozen, yellow translucent* |
| Cooling test-E1 | acetone/ACN (1 : 1, v/v) | 41.1 mg | 0.2 mL | 50 °C→5 °C | Yellow-brown oily substance |
| Cooling test-E2 | | | | 50 °C→-20 °C | A small amount of yellow-brown oily substance |
| Cooling test-B. | Acetic acid | 40.4 mg of the complex remained undissolved in 1.4 mL of solvent; After stirring at 50 °C for about 7 days, the obtained solid crystal form was consistent with the starting tretinoin. | | | |

| | | | | | |
|---|---|---|---|---|---|
| 50 °C→5 °C: The resulting clear solution was equilibrated at 50 °C for 30 min, then cooled to 5 °C within 450 min, and equilibrated at 5 °C for 3 days. 50 °C→-20 °C: The clear solution obtained at 50 °C was quickly moved to -20 °C environment and equilibrated at -20 °C for 3 days. *: Melting immediately upon taking out. | | | | | |

### 5.5 Liquid nitrogen freezing test

About 50 mg of 2[DEA][RA] was scooped using a weighing spoon, and the spoon along with the sample was placed in liquid nitrogen (the sample was a reddish-brown transparent liquid before freezing). After freezing for about 1 min, the sample was taken out, yielding a reddish-brown transparent solid (hard and brittle). But upon rewarming (room temperature: about 22 °C), the sample melted immediately (reverting to a reddish-brown transparent liquid), and no solid form substance was observed. The experimental process is as shown in FIG. 12.

### 5.6 Anti-solvent addition test

The corresponding volume of good solvent was added to an appropriate amount of 2[DEA][RA] to obtain a clear solution. If there were two corresponding anti-solvents, the clear solution was divided into two parts. At room temperature (about 22 °C), the anti-solvent was slowly added to the clear solution with stirring until solid precipitation was observed (the maximum volume of anti-solvent added was 1.5 mL). If the solid precipitation was observed, the addition of anti-solvent was stopped. After about 4 days of stirring at room temperature, a clear solution was obtained or oiling-out phenomenon was observed, without solid form substance obtained. The results are shown in the table below:

**Table E-4 Anti-solvent addition test**

| No. | Good solvent | Mass | Volume (good solvent) | Anti-solvent | Volume (anti-solvent) | Results |
|---|---|---|---|---|---|---|
| Anti-solvent addition test-A | DMSO | 20.1 mg | 0.05 mL | ACN | 1.5 mL | Clear |
| Anti-solvent addition test-B 1 | n-propan ol | 40.4 mg | 0.3 mL | ACN | 1.5 mL | Clear |
| Anti-solvent addition test-B2 | | | | n-heptane | 1.5 mL | Clear |
| Anti-solvent addition test-C 1 | acetone | 39.4 mg | 0.1 mL | ACN | 0.15 mL | Oiling-out |
| Anti-solvent addition test-C2 | | | | n-heptane | 0.15 mL | Oiling-out |
| Anti-solvent addition test-D1 | THF | 41.8 mg | 0.1 mL | ACN | 1.5 mL | Clear |
| Anti-solvent addition test-D2 | | | | n-heptane | 0.05 mL | Oiling-out |
| Anti-solvent addition test-E1 | IPA | 39.6 mg | 0.2 mL | ACN | 1.5 mL | Clear |
| Anti-solvent addition test-E2 | | | | n-heptane | 0.85 mL | Oiling-out |

### 5.7 Summary of crystallization induction test

A total of 49 crystallization induction experiments (including evaporation, stirring, cooling and anti-solvent addition, liquid nitrogen freezing) were performed starting with tretinoin-diethanolamine complexes. The reaction solvents covered different types of single solvent or binary solvent combinations, and the reaction temperatures ranged from -20 °C to 50 °C (liquid nitrogen freezing test temperature at -196 °C). No solid form substance was obtained in all experiments. The results show that it is difficult for the tretinoin-diethanolamine complexes to exist in a solid form, that is to say, the liquid form is its stable form at ambient temperature.

### Example 6 Study on prescriptions of compositions containing tretinoin-alcoholamine compound complexes

### 6.1 Study on solubility of the tretinoin-alcoholamine compound complex in different medium systems

| Oily mediums | 2[DEA][RA] | 2[DEA][RA]+water (w/w, 1 : 1) | 2[DEA][RA]+ethanol (w/w, 1 : 1) |
|---|---|---|---|
| Medium-chain triglyceride | Insoluble | Insoluble, layered | Soluble |
| Isopropyl myristate | Insoluble | Insoluble, layered | Soluble |
| Soybean oil | Insoluble | Insoluble, layered | Soluble |
| Laurocapram | Insoluble | Insoluble, layered | Soluble |

The experiments show that the tretinoin-alcoholamine compound complex exhibits poor solubility in the above oily mediums. Therefore, a further study of suitable preparation systems is required.

### 6.2 Gel matrix study

### 6.2.1 PVA matrix-prescription:

| Ingredient/Manufacturer | | Prescribed amount |
|---|---|---|
| PVA(MW13000-23000) | Sigma-Aldrich | 10% |
| Glycerol | Sinopharm Chemical Reagent Co., Ltd., China | 10% |
| Benzyl alcohol | Sinopharm Chemical Reagent Co., Ltd., China | 1% |
| Purified water | Homemade | 79% |
| Total | | 100% |

About 80% of the prescribed amount of water was taken, and heated to 85 °C to 90 °C. PVA was weighed, added to the water, and a resulting mixture was stirred under heating in a water bath at 85 °C until uniform dispersion and swelling were achieved; the prescribed amounts of glycerol and benzyl alcohol were added thereto, and water was supplemented to the prescribed amount; and after cooling to room temperature, the resulting mixture was frozen at -20 °C for 4 h, and then taken out and thawed at ambient temperature. This freezing-thawing cycle was repeated twice to obtain the PVA matrix.

### 6.2.2 H-HPC matrix-prescription:

| Ingredient/Manufacturer | | Prescribed amount |
|---|---|---|
| H-HPC | Ashland | 5% |
| Glycerol | Sinopharm Chemical Reagent Co., Ltd., China | 10% |
| Benzyl alcohol | Sinopharm Chemical Reagent Co., Ltd., China | 1% |
| Purified water | Homemade | 84%% |
| Total | | 100% |

About 80% of the prescribed amount of water was taken and heated to about 40 °C; H-HPC was weighed, added to the water, and a resulting mixture was stirred at ambient temperature until uniform dispersion and swelling were achieved; and the prescribed amounts of glycerol and benzyl alcohol were added, water was supplemented to the prescribed amount, and stirring was performed until uniform to obtain the H-HPC matrix.

### 6.2.3 HPMC matrix-prescription:

| Ingredient/Manufacturer | | Prescribed amount |
|---|---|---|
| HPMC K15M | Ashland | 5% |
| Glycerol | Sinopharm Chemical Reagent Co., Ltd., China | 10% |
| Benzyl alcohol | Sinopharm Chemical Reagent Co., Ltd., China | 1% |
| Purified water | Homemade | 84% |
| Total | | 100% |

HPMC was weighed, added to about 80% of the prescribed amount of water, and a resulting mixture was stirred at ambient temperature until uniform dispersion and swelling were achieved; and the prescribed amounts of glycerol and benzyl alcohol were added, water was supplemented to the prescribed amount, and stirring was performed until uniform to obtain the HPMC matrix.

### 6.2.4 Colloidal silica-propylene glycol matrix-prescription:

| Ingredient/Manufacturer | | Prescribed amount |
|---|---|---|
| Colloidal silica | EVONIK | 5% |
| Tween 80 | Sinopharm Chemical Reagent Co., Ltd., China | 1% |
| Propylene glycol | Aladdin | 10% |
| Benzyl alcohol | Sinopharm Chemical Reagent Co., Ltd., China | 1% |
| Purified water | Homemade | 83%% |
| Total | | 100% |

Various materials were weighed, added to water, and a resulting mixture was stirred at ambient temperature until uniform dispersion; and homogenization was performed at 10000 rpm for 1 min using a high-shear homogenizer to obtain the product.

### 6.2.5 Polycarbophil matrix-prescription:

| Ingredient/Manufacturer | | Prescribed amount |
|---|---|---|
| Polycarbophil AA-1 | Lubrizol | 3% |
| PVP K30 | Sinopharm Chemical Reagent Co., Ltd., China | 6% |
| Triethanolamine | Sinopharm Chemical Reagent Co., Ltd., China | 3% |
| Glycerol | Sinopharm Chemical Reagent Co., Ltd., China | 5% |
| Propylene glycol | Aladdin | 30% |
| Benzyl alcohol | Sinopharm Chemical Reagent Co., Ltd., China | 1% |
| Purified water | Homemade | 52% |
| Total | | 100% |

Triethanolamine was dissolved in water, PVP K30 was added, and a resulting mixture was stirred until uniform dispersion; benzyl alcohol, propylene glycol and glycerol were added thereto, and stirring was performed until uniformly mixed; the polycarbophil after passing through a 35-mesh sieve was added to the system, and stirring was performed until uniform dispersion; and homogenization was performed at 1000 rpm for 2 min using a high-shear homogenizer to obtain the Polycarbophil matrix.

### 6.2.6 PEG ointment matrix-prescription

| Ingredient/Manufacturer | | Proportion |
|---|---|---|
| 2[DEA][RA] | Homemade | 0.085% (containing 0.05% of tretinoin) |
| Polyethylene glycol 400 | Shandong Ruisheng Pharmaceutical Excipient Co., Ltd.,China | 67.915% |
| Polyethylene glycol 3350 | Union Carbide | 32% |
| Total | | 100% |

Polyethylene glycol 400 and polyethylene glycol 3350 were placed in a 250 mL beaker, and stirred at 100 rpm in a water bath at 60 °C for melting and mixing. The raw material was weighted in the dark, and added to the matrix solution obtained above, and stirring was continued until dissolved. The resulting system was cooled in a water bath at 40 °C, then vacuumized, and then cooled to about 40 °C. Homogenization was performed at 3000 rpm for 5 min using a homogenizer, followed by natural cooling to obtain the PEG ointment matrix.

### 6.2.7 Carbopol 980 matrix-prescription

| Ingredient | Manufacturer | Prescribed amount |
|---|---|---|
| Carbopol 980 | Lubrizol | 2% |
| Glycerol | Sinopharm Chemical Reagent Co., Ltd., China | 10% |
| Benzyl alcohol | Sinopharm Chemical Reagent Co., Ltd., China | 1% |
| Purified water | Homemade | 87% |
| Triethanolamine | Sinopharm Chemical Reagent Co., Ltd., China | In an amount to adjust pH to 5.0 to 5.5 |
| Total | | 100% |

Carbopol 980 was weighed, added to about 60% of the prescribed amount of water, and a resulting mixture was stirred at ambient temperature until uniform dispersion; the prescribed amounts of glycerol and benzyl alcohol were added thereto, and stirring was performed until uniform; an appropriate amount of triethanolamine was added thereto, such that the pH value of the resulting system was adjusted to 5.0 to 5.5 for gelling, and purified water was supplemented to the prescribed amount; and stirring was continued for 20 min using an anchor stirrer to obtain the Carbopol 980 matrix.

### 6.2.8 Carbopol ETD2020 matrix-prescription

| Ingredient | Manufacturer | Prescribed amount |
|---|---|---|
| Carbopol ETD2020 | Lubrizol | 2% |

| | | |
|---|---|---|
| Ethanol | Sinopharm Chemical Reagent Co., Ltd., China | 5% |
| Glycerol | Sinopharm Chemical Reagent Co., Ltd., China | 10% |
| Benzyl alcohol | Sinopharm Chemical Reagent Co., Ltd., China | 1% |
| Purified water | Homemade | 82% |
| Triethanolamine | Sinopharm Chemical Reagent Co., Ltd., China | In an amount to adjust pH value to 5.0 to 5.5 |
| Total | | 100% |

Carbopol ETD2020 was weighed, added to about 60% of the prescribed amount of water, and a resulting mixture was stirred at ambient temperature until uniform dispersion; the prescribed amounts of ethanol, glycerol and benzyl alcohol were added thereto, and stirring was performed until uniform; an appropriate amount of triethanolamine was added thereto, such that the pH value of the resulting system was adjusted to 5.0 to 5.5 for gelling, and purified water was supplemented to the prescribed amount; and stirring was continued for 20 min using an anchor stirrer to obtain the Carbopol ETD2020 matrix.

### 6.2.9 Evaluation of different matrix prescriptions

**Table F**

| | Matrix material | Evaluation of matrix |
|---|---|---|
| 1 | PVA matrix | Formed as a pale yellow gel, which could form a film upon application and exhibit poor skin feel. |
| 2 | H-HPC | Formed as a transparent gel, which had a low mechanical strength and could form a film upon application. |
| 3 | HPMC | Formed as a transparent gel, which had a low mechanical strength and exhibited sticky feel upon application. |
| 4 | Colloidal silica-propylene glycol matrix | Formed as a translucent thixotropic gel, which was liquefied upon application and left white marks after drying, adversely affecting the aesthetics. |
| 5 | Polycarbophil matrix | Formed as a transparent adhesive gel, which exhibited moderate skin feel. However, polycarbophil is rarely used alone as a gel matrix, is costly, and lacks significant advantages, making it a non-preferred choice. |
| 6 | PEG ointment matrix | Formed as a white ointment, which exhibited strong greasy feel upon application. |
| 7 | Carbopol 980 matrix | Formed as a transparent gel, which had a certain thixotropy and exhibited cooling feeling after application |
| 8 | Carbopol ETD2020 matrix | Formed as a transparent gel, which had a certain thixotropy and exhibited cooling feeling after application |

On the basis of the above comparison results, carbomer was preferred as the semi-solid matrix material.

### 6.3 Study on compatibility of gel matrix and complex

**Table G**

| | Prescription G-1 | Prescription G-2 | Prescription G-3 |
|---|---|---|---|
| Complex 2[DEA][RA] (amount) | 12% | 5% | 0.17% |
| Carbopol 980 (amount) | 1% | 1% | 1% |
| NaOH | Q.S. | Q.S. | Q.S. |
| Water | 87% | 94% | 98.83% |
| State and compatibility of preparation | Clear gel, compatible | Yellow solid precipitated | Yellow solid precipitated |

0.17% (0.1% in terms of RA) of 2[DEA][RA] complex was added to the Carbopol 980 gel matrix, and the precipitation of yellow solids from the reddish-brown tretinoin complex was observed, that was to say, the complex was destabilized by the moisture in the matrix.

Be limited to the amount of tretinoin, the tretinoin complex was destabilized in a gel matrix with a high water content, resulting in tretinoin precipitation. Therefore, the Carbopol 980 gel matrix with a high water content was incompatible with the tretinoin complex. Moreover, tretinoin in the particulate form was not conducive to transdermal absorption. Therefore, it was necessary to further add additional auxiliary materials to the prescription to facilitate compatibility with the complex, to avoid tretinoin precipitation, thereby forming a stable semi-solid preparation.

### 6.4 Semi-solid preparation containing tretinoin-alcoholamine compound complex

### 6.4.1 Cream:

### Prescription 1

| Ingredient/Manufacturer | | | Proportion |
|---|---|---|---|
| Aqueous phase | Carbopol | Lubrizol | 1% |
| | Sodium metabisulfite | Hunan Er-kang Pharmaceutical Co., Ltd., China | 0.2% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 96.59% |
| Oil phase | 2[DEA][RA] | Homemade | 0.17% (containing 0.1% of tretinoin) |
| | Polyoxyl 54 hydrogenated castor oil | Nanjing Well Pharmaceutical Group Co., Ltd., China | 2% |
| pH adjuster | NaOH | Sinopharm Chemical Reagent Co., Ltd., China | In an amount to adjust pH to 5.5 |
| Total | | | 100% |

### Prescription 2

| Ingredient/Manufacturer | | | Proportion |
|---|---|---|---|
| Aqueous phase | Carbopol | Lubrizol | 1% |
| | Sodium metabisulfite | Hunan Er-kang Pharmaceutical Co., Ltd., China | 0.2% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 76.59% |
| Oil phase | 2[DEA][RA] | Homemade | 0.17% (containing 0.1% of tretinoin) |
| | Glyceryl monocaprylate | IMCD (China) Co., Ltd. | 2% |
| | Polyoxyl 54 hydrogenated castor oil | Nanjing Well Pharmaceutical Group Co., Ltd., China | 10% |
| | Glycerol | Shandong Ruisheng Pharmaceutical Excipient Co., Ltd., China | 10% |
| pH adjuster | NaOH | Sinopharm Chemical Reagent Co., Ltd., China | In an amount to adjust pH to 5.5 |
| Total | | | 100% |

**Cream process of Prescriptions 1-2:** Polyoxyl 54 hydrogenated castor oil was melted by heating at 60 °C, and cooled to 40 °C, and the remaining raw materials and auxiliary materials of the oil phase were added thereto and mixed until dissolved; the materials of the aqueous phase were mixed, and Carbopol was added thereto until uniform dispersion; the two phases were mixed, and homogenization was performed at 1000 rpm for 2 min using a homogenizer; an appropriate amount of NaOH was added to adjust the pH value to 5.0 to 5.5; and the resulting material was filled into aluminum-plastic composite tubes to obtain the product.

### 6.4.2. Emulgel

### Prescription 3

| Ingredient/Manufacturer | | | Proportion |
|---|---|---|---|
| Aqueous phase | Carbopol 980 | Lubrizol | 0.2% |
| | Pemulen TR-2 NF | Lubrizol | 0.3% |
| | Tween 80 | Sigma | 0.3% |
| | Water | Homemade | 67.86% |
| Oil phase | 2[DEA][RA] | Homemade | 0.17% (containing 0.1% of tretinoin) |
| | Ethanol | Sinopharm Chemical Reagent Co., Ltd., China | 2.5% |
| | Octadecanol (stearyl alcohol) | Sinopharm Chemical Reagent Co., Ltd., China | 5% |
| | Glycerol | Shandong Ruisheng Pharmaceutical Excipient Co., Ltd., China | 10% |
| | Dibutyl hydroxytoluene | Jiangxi Alpha Hi-tech Pharmaceutical Co., Ltd., China | 0.1% |
| | Phenoxyethanol | Sinopharm Chemical Reagent Co., Ltd., China | 1% |
| | Isopropyl myristate | Shanghai Macklin Biochemical Technology Co., Ltd., China | 12.57% |
| pH adjuster | Triethanolamine | Sinopharm Chemical Reagent Co., Ltd., China | In an amount to adjust pH to 5.5 |
| Total | | | 100% |

### Prescription 4

| Ingredient/Manufacturer | | | Proportion |
|---|---|---|---|
| Aqueous phase | Carbopol ETD2020 | Lubrizol | 0.2% |
| | Pemulen TR-1 NF | Lubrizol | 0.3% |
| | Tween 80 | Sigma | 0.3% |
| | Water | Homemade | 67.86% |
| Oil phase | 2[DEA][RA] | Homemade | 0.17% (containing 0.1% of tretinoin) |
| | Ethanol | Sinopharm Chemical Reagent Co., Ltd., China | 2.5% |
| | Octadecanol (stearyl alcohol) | Sinopharm Chemical Reagent Co., Ltd., China | 5% |
| | Glycerol | Shandong Ruisheng Pharmaceutical Excipient Co., Ltd., China | 10% |
| | Dibutyl hydroxytoluene | Jiangxi Alpha Hi-tech Pharmaceutical Co., Ltd., China | 0.1% |
| | Benzyl alcohol | Sinopharm Chemical Reagent Co., Ltd., China | 1% |
| | Isopropyl myristate | Shanghai Macklin Biochemical Technology Co., Ltd., China | 12.57% |
| pH adjuster | Triethanolamine | Sinopharm Chemical Reagent Co., Ltd., China | In an amount to adjust pH to 5.5 |
| Total | | | 100% |

**Emulgel process of Prescriptions 3-4:** The prescribed amounts of all auxiliary materials of the aqueous phase were weighed, and uniformly mixed, and the resulting solution was placed in a water bath at 40 °C for later use. The prescribed amounts of 2[DEA][RA] and ethanol were weighed, and mixed. Isopropyl myristate and benzyl alcohol (or phenoxyethanol) were weighed and added thereto, and mixing was performed until uniform. Octadecanol and dibutyl hydroxytoluene were weighed and added to the oil phase, then heated in a water bath at 60 °C until dissolved, and a resulting mixture was then cooled to 40 °C. In a water bath at 40 °C, the oil phase was added to the aqueous phase, and homogenization was performed for 3 min using a high-shear homogenizer; an appropriate amount of triethanolamine was added thereto to adjust the pH value of the resulting mixture to 5.0 to 5.5; mechanical stirring (four-blade or anchor) was performed for mixing until an opaque semi-solid emulgel was formed. The emulgel was filled into aluminum-plastic composite tubes to obtain the product.

### 6.4.3 Gel

### Prescription 5

| Ingredient/Manufacturer | | | Proportion |
|---|---|---|---|
| Aqueous phase | Carbopol | Lubrizol | 1% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 81.59% |
| Oil phase | 2[DEA][RA] | Homemade | 0.17% (containing 0.1% of tretinoin) |
| | Dibutyl hydroxytoluene | Jiangxi Alpha Hi-tech Pharmaceutical Co., Ltd., China | 0.2% |
| | Laurocapram | Shandong Ruisheng Pharmaceutical Excipient Co., Ltd., China | 2% |
| | Propylene glycol | Shandong Ruisheng Pharmaceutical Excipient Co., Ltd., China | 15% |
| pH adjuster | NaOH | Sinopharm Chemical Reagent Co., Ltd., China | In an amount to adjust pH to 5.5 |
| Total | | | 100% |

### Prescription 6

| Ingredient/Manufacturer | | | Proportion |
|---|---|---|---|
| Aqueous phase | Carbopol | Lubrizol | 1% |
| | Sodium metabisulfite | Hunan Er-kang Pharmaceutical Co., Ltd., China | 0.2% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 81.59% |
| | | | |
| Oil phase | 2[DEA][RA] | Homemade | 0.17% (containing 0.1% of tretinoin) |
| | Laurocapram | Shandong Ruisheng Pharmaceutical Excipient Co., Ltd., China | 2% |
| | Propylene glycol | Shandong Ruisheng Pharmaceutical Excipient Co., Ltd., China | 15% |
| pH adjuster | NaOH | Sinopharm Chemical Reagent Co., Ltd., China | In an amount to adjust pH to 5.5 |
| Total | | | 100% |

### Prescription 7

| Ingredient/Manufacturer | | | Proportion |
|---|---|---|---|
| Aqueous phase | Carbopol | Lubrizol | 1% |
| | Vitamin C | Sinopharm Chemical Reagent Co., Ltd., China | 0.2% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 81.59% |
| Oil phase | 2[DEA][RA] | Homemade | 0.17% (containing 0.1% of tretinoin) |
| | Laurocapram | Shandong Ruisheng Pharmaceutical Excipient Co., Ltd., China | 2% |
| | Propylene glycol | Shandong Ruisheng Pharmaceutical Excipient Co., Ltd., China | 15% |
| pH adjuster | NaOH | Sinopharm Chemical Reagent Co., Ltd., China | In an amount to adjust pH to 5.5 |
| Total | | | 100% |

### Prescription 8

| Ingredient/Manufacturer | | | Proportion |
|---|---|---|---|
| Aqueous phase | Carbopol | Lubrizol | 1% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd. | 0.02% |
| | Water | Homemade | 81.59% |
| Oil phase | 2[DEA][RA] | Homemade | 0.17% (containing 0.1% of tretinoin) |
| | Dibutyl hydroxytoluene | Jiangxi Alpha Hi-tech Pharmaceutical Co., Ltd., China | 0.2% |
| | Glyceryl monocaprylate | IMCD (China) Co., Ltd. | 2% |
| | Propylene glycol | Shandong Ruisheng Pharmaceutical Excipient Co., Ltd., China | 15% |
| pH adjuster | NaOH | Sinopharm Chemical Reagent Co., Ltd., China | In an amount to adjust pH value to 5.5 |
| Total | | | 100% |

### Prescription 9

| Ingredient/Manufacturer | | | Proportion |
|---|---|---|---|
| Aqueous phase | Carbopol | Lubrizol | 1% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 63.59% |
| Glycerol phase | 2[DEA][RA] | Homemade | 0.17% (containing 0.1% of tretinoin) |
| | Dibutyl hydroxytoluene | Jiangxi Alpha Hi-tech Pharmaceutical Co., Ltd., China | 0.2% |
| | Diethylene glycol monoethyl ether | Gattefosse (Shanghai) Trading Co., Ltd., China | 20% |
| | Glycerol | Shandong Ruisheng Pharmaceutical Excipient Co., Ltd., China | 15% |
| pH adjuster | NaOH | Sinopharm Chemical Reagent Co., Ltd., China | In an amount to adjust pH value to 5.5 |
| Total | | | 100% |

### Prescription 10

| Ingredient/Manufacturer | | | Proportion |
|---|---|---|---|
| Aqueous phase | Carbopol | Lubrizol | 1% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 73.59% |
| Oil phase | 2[DEA][RA] | Homemade | 0.17% (containing 0.1% of tretinoin) |
| | Dibutyl hydroxytoluene | Jiangxi Alpha Hi-tech Pharmaceutical Co., Ltd., China | 0.2% |
| | Polyethylene glycol glyceryl caprylate/caprate | Gattefosse (Shanghai) Trading Co., Ltd., China | 10% |
| | Glycerol | Shandong Ruisheng Pharmaceutical Excipient Co., Ltd., China | 15% |
| pH adjuster | NaOH | Sinopharm Chemical Reagent Co., Ltd., China | In an amount to adjust pH value to 5.5 |
| Total | | | 100% |

### Prescription 11

| Ingredient/Manufacturer | | | Proportion |
|---|---|---|---|
| Aqueous phase | Carbopol | Lubrizol | 1% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 81.50% |
| Oil phase | 2[DEA][RA] | Homemade | 0.26% (containing 0.15% of tretinoin) |
| | Dibutyl hydroxytoluene | Jiangxi Alpha Hi-tech Pharmaceutical Co., Ltd., China | 0.2% |
| | Laurocapram | Shandong Ruisheng Pharmaceutical Excipient Co., Ltd., China | 2% |
| | Propylene glycol | Shandong Ruisheng Pharmaceutical Excipient Co., Ltd., China | 15% |
| pH adjuster | NaOH | Sinopharm Chemical Reagent Co., Ltd., China | In an amount to adjust pH value to 5.5 |
| Total | | | 100% |

### Prescription 12

| Ingredient/Manufacturer | | | Proportion |
|---|---|---|---|
| Aqueous phase | Carbopol | Lubrizol | 1% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 81.67% |
| Oil phase | 2[DEA][RA] | Homemade | 0.09% (containing 0.05% of tretinoin) |
| | Dibutyl hydroxytoluene | Jiangxi Alpha Hi-tech Pharmaceutical Co., Ltd., China | 0.2% |
| | Laurocapram | Shandong Ruisheng Pharmaceutical Excipient Co., Ltd., China | 2% |
| | Propylene glycol | Shandong Ruisheng Pharmaceutical Excipient Co., Ltd., China | 15% |
| pH adjuster | NaOH | Sinopharm Chemical Reagent Co., Ltd., China | In an amount to adjust pH value to 5.5 |
| Total | | | 100% |

### Prescription 13

| Ingredient/Manufacturer | | | Proportion |
|---|---|---|---|
| Aqueous phase | Carbopol | Lubrizol | 1% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 96.59% |
| Oil phase | 2[DEA][RA] | Homemade | 0.17% (containing 0.1% of tretinoin) |
| | Dibutyl hydroxytoluene | Jiangxi Alpha Hi-tech Pharmaceutical Co., Ltd., China | 0.2% |
| | laurocapram | Shandong Ruisheng Pharmaceutical Excipient Co., Ltd., China | 2% |

| | | | |
|---|---|---|---|
| pH adjuster | NaOH | Sinopharm Chemical Reagent Co., Ltd., China | In an amount to adjust pH value to 5.5 |
| Total | | | 100% |

### Prescription 14

| Ingredient/Manufacturer | | | Proportion |
|---|---|---|---|
| Aqueous phase | Carbopol | Lubrizol | 1% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 81.59% |
| Oil phase | 2[DEA][RA] | Homemade | 0.17% (containing 0.1% of tretinoin) |
| | Dibutyl hydroxytoluene | Jiangxi Alpha Hi-tech Pharmaceutical Co., Ltd., China | 0.2% |
| | Laurocapram | Shandong Ruisheng Pharmaceutical Excipient Co., Ltd., China | 2% |
| | Glycerol | Shandong Ruisheng Pharmaceutical Excipient Co., Ltd., China | 15% |
| pH adjuster | NaOH | Sinopharm Chemical Reagent Co., Ltd., China | In an amount to adjust pH value to 5.5 |
| Total | | | 100% |

**Gel process of Prescription 5 to Prescription 14:** 60% of the prescribed amount of water was weighed, a preservative was added thereto, and a resulting mixture was stirred until dissolved. Carbomer was added thereto, and stirring was performed at a high speed of 200 rpm for 5 min until uniform dispersion; stirring was performed at a low speed of 50 rpm for 50 min to hydrate the carbomer; and 2[DEA][RA] was added to the oil phase auxiliary materials, and stirring was performed until uniformly mixed to form a homogeneous oil phase. The aqueous phase was added to the oil phase, and homogenization was performed at 5000 rpm for 3 min using a high-shear homogenizer. An appropriate amount of NaOH was added thereto to adjust the pH value of a resulting mixture to 5.0 to 5.5, and mechanical stirring was performed at a low speed for 30 min to form a homogeneous gel. The gel was filled into aluminum-plastic composite tubes to obtain the product.

### 6.4.4 Control preparation

### Control emulgel-Prescription 15

| Ingredient/Manufacturer | | | Proportion |
|---|---|---|---|
| Aqueous phase | Carbopol ETD2020 | Lubrizol | 0.2% |
| | Pemulen TR-1 NF | Lubrizol | 0.3% |
| | Tween 80 | Sigma | 0.3% |
| | Water | Homemade | 67.93% |
| Oil phase | Tretinoin | Shanghai Macklin Biochemical Technology Co., Ltd., China | 0.1% |
| | Ethanol | Sinopharm Chemical Reagent Co., Ltd., China | 2.5% |
| | Octadecanol (stearyl alcohol) | Sinopharm Chemical Reagent Co., Ltd., China | 5% |
| | Glycerol | Shandong Ruisheng Pharmaceutical Excipient Co., Ltd., China | 10% |
| | Dibutyl hydroxytoluene | Jiangxi Alpha Hi-tech Pharmaceutical Co., Ltd., China | 0.1% |
| | Benzyl alcohol | Sinopharm Chemical Reagent Co., Ltd., China | 1% |
| | Isopropyl myristate | Shanghai Macklin Biochemical Technology Co., Ltd., China | 12.57% |

| | | | |
|---|---|---|---|
| pH adjuster | Triethanolamine | Sinopharm Chemical Reagent Co., Ltd., China | In an amount to adjust pH value to 5.5 |
| Total | | | 100% |

**Emulgel process of Prescription 15:** The prescribed amounts of all aqueous phase auxiliary materials were weighed, and mixed uniformly; the resulting solution was kept in a water bath at 40 °C for later use. The prescribed amounts of tretinoin and ethanol were weighed and mixed. Isopropyl myristate and benzyl alcohol were weighed and added thereto, and mixing was performed until uniform. Octadecanol and dibutyl hydroxytoluene were weighed and added to the oil phase, a resulting mixture was then heated in a water bath at 60 °C until dissolved, and a resulting mixture was then cooled to 40 °C. In a water bath at 40 °C, the oil phase was added to the aqueous phase, and homogenization was performed for 3 min using a high-shear homogenizer; an appropriate amount of triethanolamine was added thereto to adjust the pH value to 5.0 to 5.5; mechanical stirring (four-blade or anchor) was performed for mixing until an opaque semi-solid emulgel was formed. The emulgel was filled into aluminum-plastic composite tubes to obtain the product.

### Control gel-Prescription 16

| Ingredient/Manufacturer | | | Proportion |
|---|---|---|---|
| Aqueous phase | Carbopol | Lubrizol | 1% |
| | Sodium methylparaben | Jiangxi Alpha Hi-tech Pharmaceutical Co., Ltd., China | 0.02% |
| | Sodium propylparaben | Jiangxi Alpha Hi-tech Pharmaceutical Co., Ltd., China | 0.02% |
| | Water | Homemade | 81.66% |

| | | | |
|---|---|---|---|
| Oil phase | Tretinoin | Homemade | 0.1% |
| | dibutyl hydroxytoluene | Jiangxi Alpha Hi-tech Pharmaceutical Co., Ltd., China | 0.2% |
| | Laurocapram | Shandong Ruisheng Pharmaceutical Excipient Co., Ltd., China | 2% |
| | Propylene glycol | Shandong Ruisheng Pharmaceutical Excipient Co., Ltd., China | 15% |
| pH adjuster | NaOH | Sinopharm Chemical Reagent Co., Ltd., China | In an amount to adjust pH to 5.5 |
| Total | | | 100% |

### Gel process of Prescription 16:

60% of the prescribed amount of water was weighed, auxiliary materials such as a preservative were added, and stirring was performed until dissolved. Carbomer was added thereto, and stirring was performed at a high speed of 200 rpm for 5 min until uniform dispersion; stirring was performed at a low speed of 50 rpm for 50 min to hydrate the carbomer; tretinoin was added to the oil phase auxiliary materials, and stirring was performed until uniformly mixed to form a homogeneous oil phase. The aqueous phase was added to the oil phase, and homogenization was performed at 5000 rpm for 3 min using a high-shear homogenizer. An appropriate amount of NaOH was added to adjust the pH value of the resulting mixture to 5.0 to 5.5, and mechanical stirring was performed at a low speed for 30 min to form a homogeneous gel. The gel was filled into aluminum-plastic composite tubes to obtain the product.

### 6.4.5 Stability of the preparations of the present disclosure

**Table H**

| Prescription | Stability of preparation |
|---|---|
| 2[DEA][RA] ointment (Prescription 2) | The preparation was stored at room temperature for 180 days, without significant change in properties and no graininess feel upon application. Through observation by microscope, minor solid crystal precipitation was observed, i.e., partial tretinoin precipitated. |
| 2[DEA][RA] emulgel (Prescription 4) | The preparation was stored at room temperature for 180 days, without significant change in properties and no graininess feel upon application. Through observation by microscope, the adduct was present as small droplets, without precipitation observed. |
| Tretinoin emulgel (Prescription 15) | The preparation was stored at room temperature for 180 days, without significant change in properties and no graininess feel upon application. Through observation by microscope, needle-like crystal was observed, i.e., tretinoin precipitation. |
| 2[DEA][RA] gel (Prescription 5) | The preparation was stored at room temperature for 180 days, without significant change in properties and no graininess feel upon application. Through observation by microscope, the adduct was presented as small droplets, without precipitation observed. |
| Tretinoin gel (Prescription 16) | The preparation was stored at room temperature for 180 days, without significant change in properties and no graininess feel upon application. Through observation by microscope, needle-like crystal was observed, i.e., tretinoin precipitation. |

### 6.4.6 Transdermal permeation test results of the preparations of the present disclosure

**Table I Transdermal permeation test results**

| Sample | Permeation amount per unit area, ng/cm² | | | | | |
|---|---|---|---|---|---|---|
| | 1 | 2 | 3 | 4 | Mean | RSD% |
| Prescription 4 | 280.87 | 202.36 | 299.53 | 183.32 | 241.52 | 23.7 |
| Prescription 15 | 27.06 | 20.61 | 16.59 | 17.83 | 20.52 | 22.8 |
| Prescription 5 | 270.42 | 292.78 | 168.20 | 216.42 | 236.96 | 23.6 |
| Prescription 16 | 15.09 | 28.67 | 24.55 | 11.43 | 19.94 | 40.3 |
| Diwei (commercial preparation) | 20.64 | 19.58 | 21.91 | 29.64 | 22.94 | 19.9 |
| Baoyining (commercial preparation) | 62.71 | 49.08 | 38.90 | 43.92 | 48.65 | 21.1 |

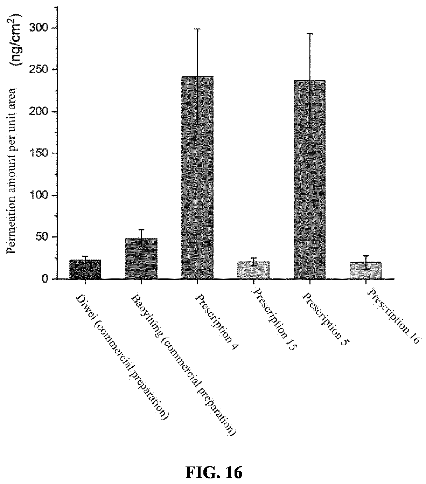

The results (see FIG. 16) show that the preparations (Prescription 4, Prescription 5) of the present disclosure exhibit significant permeation-promoting effects, where the skin retention of the 2[DEA][RA] emulgel preparation (Prescription 4) is 11.8-fold higher than that of the tretinoin emulgel preparation (Prescription 15); the skin retention of the 2[DEA][RA] gel preparation (Prescription 5) is 11.9-fold higher than that of the tretinoin gel preparation (Prescription 16); and compared with the commercial preparations, Prescription 4 and Prescription 5 achieve 4.9- to 10.5-fold greater skin retention than those of the commercial preparations.

The embodiments of the present disclosure are illustrated as above. However, the present disclosure is not limited to the above-mentioned embodiments. Any modifications, equivalent substitutions, improvements, etc. made within the spirit and principles of the present disclosure shall be included within the scope of the present disclosure.

## Claims

1. A composition, comprising the components of:
(a) a tretinoin-alcoholamine compound complex;
(b) a gel matrix; and
(c) an oil phase component;
wherein the tretinoin-alcoholamine compound complex is composed of tretinoin and an alcoholamine compound.

2. The composition as claimed in claim 1, wherein the alcoholamine compound is represented by formula I: 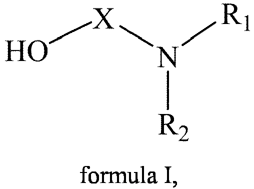
in formula I, X is selected from C₁₋₆ alkyl, and preferably is selected from the group consisting of -CH₂-CH₂-, -CH₂-CH₂-CH₂-, and 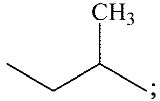
R₁ and R₂ each are independently selected from the group consisting of H, C₁₋₆ alkyl, and C₁₋₆ alkyl-OH;
preferably, the formula I is selected from the group consisting of diethanolamine, triethanolamine, monoethanolamine (2-hydroxyethylamine), N-methyldiethanolamine, n-propanolamine, isopropanolamine, diisopropanolamine, triisopropanolamine, n-butanolamine, dibutanolamine, and isobutanolamine; and more preferably, a molar ratio of the alcoholamine compound to the tretinoin ranges from 1 : 1 to 100 : 1.

3. The composition as claimed in claim 1 or 2, wherein the (c) oil phase component is at least one selected from the group consisting of (c1) a greasy matrix, (c2) a co-solvent, and (c3) a penetration enhancer.

4. The composition as claimed in any one of claims 1-3, wherein the composition is selected from the group consisting of the following formulation I to formulation V:
formulation I:
(a) the tretinoin-alcoholamine compound complex;
(b) the gel matrix; and
(d) an emulsifier;
formulation II:
(a) the tretinoin-alcoholamine compound complex;
(b) the gel matrix;
(c2) a co-solvent;
(c3) a penetration enhancer; and
(d) an emulsifier;
formulation III:
(a) the tretinoin-alcoholamine compound complex;
(b) the gel matrix;
(c1) a greasy matrix;
(c2) a co-solvent; and
(d) an emulsifier;
formulation IV:
(a) the tretinoin-alcoholamine compound complex;
(b) the gel matrix;
(c2) a co-solvent; and
(c3) a penetration enhancer;
and formulation V:
(a) the tretinoin-alcoholamine compound complex;
(b) the gel matrix; and
(c3) a penetration enhancer.

5. The composition as claimed in any one of claims 1-4, wherein the gel matrix is selected carbomers, such as Carbopol 980, and Carbopol ETD2020;
the co-solvent is selected from alcohols, and preferably is at least one selected from the group consisting of glycerol, ethanol, propylene glycol, and benzyl alcohol;
the emulsifier is at least one selected from the group consisting of Tween 20, Tween 40, Tween 60, Tween 80, Span 20, Span 60, Span 80, a polyoxyethylene castor oil derivative, poloxamer, Triton, polyethylene glycol glyceryl caprylate/caprate, polyethylene glycol stearate, polyethylene oxide-8 beeswax, and lauroyl macrogolglyceride;
the greasy matrix is at least one selected from the group consisting of octadecanol, hexadecanol, stearic acid, and a fatty acid ester; and
the penetration enhancer is at least one selected from the group consisting of glyceryl monocaprylate, laurocapram, diethylene glycol monoethyl ether, a polyglycerol fatty acid ester, propylene glycol monocaprylate, and polyethylene glycol glyceryl caprylate/caprate.

6. The composition as claimed in any one of claims 1-5, further comprising at least one selected from the group consisting of an antioxidant, a preservative, a pH adjuster, a metal ion complexing agent, and a rheology modifier.

7. The composition as claimed in claim 6, wherein the antioxidant is at least one selected from the group consisting of dibutyl hydroxytoluene, butylhydroxylanisole, vitamin C, vitamin E, and sodium metabisulfite;
the preservative is at least one selected from the group consisting of benzyl alcohol, phenoxyethanol, sodium benzoate, methylparaben, ethylparaben, propylparaben, sodium methylparaben, and sodium propylparaben;
the pH adjuster is at least one selected from the group consisting of triethanolamine and sodium hydroxide;
the rheology modifier is at least one selected from the group consisting of Pemulen^{™} TR-1 NF polymer, Pemulen^{™} TR-2 NF polymer, Carbopol^{®} 1342 NF polymer, and Carbopol^{®} 5984 EP polymer;
the metal ion complexing agent is at least one selected from the group consisting of ethylenediaminetetraacetic acid (EDTA), disodium ethylenediaminetetraacetic acid (EDTA-2Na), tetrasodium ethylenediaminetetraacetic acid (EDTA-4Na), disodium nitrilotriacetate, sodium tripolyphosphate, sodium hexametaphosphate, and tetrapotassium pyrophosphate; and
preferably, the composition has a pH value of 4-7.

8. The composition as claimed in any one of claims 1-7, wherein
component (a) accounts for 0.01% to 5% (w/w) of a total amount of the composition;
component (b) accounts for 0.01% to 5% (w/w) of the total amount of the composition;
component (c) accounts for 0.5% to 70.0% (w/w) of the total amount of the composition; and
component (d) accounts for 0.5% to 30.0% (w/w) of the total amount of the composition.

9. The composition as claimed in any one of claims 1-7, wherein
component (c1) accounts for 4.0%-30.0% (w/w) of a total amount of the composition;
component (c2) accounts for 0.5% to 25.0% (w/w) of the total amount of the composition; and
component (c3) accounts for 0.5% to 30.0% (w/w) of the total amount of the composition.

10. Use of the composition as claimed in any one of claims 1-9 in the manufacture of a preparation, wherein the preparation is selected from the group consisting of a pharmaceutical preparation, a cosmetic product, a care product, and a beauty product; and
preferably, the pharmaceutical preparation is used for the treatment of a skin disease, such as acne, hyperpigmentation, skin photoaging, psoriasis, ichthyosis, lichen planus, pityriasis rubra pilaris, keratosis follicularis, squamous cell carcinoma, and melanoma.
